# EUROPÄISCHE PATENTSCHRIFT

(11) **EP 1 651 614 B1**
(45) Veröffentlichungstag und Bekanntmachung des Hinweises auf die Patenterteilung: **11.07.2007**
(21) Anmeldenummer: 04740458.7
(22) Anmeldetag: 30.06.2004
(51) Int. Cl.: C07D 233/54, C07D 521/00

(54) **VERFAHREN ZUR HERSTELLUNG VON GEREINIGTEN 1,3-SUBSTITUIERTEN IMIDAZOLIUMSALZEN**
METHOD FOR THE PRODUCTION OF PURIFIED 1,3-SUBSTITUTED IMIDAZOLIUM SALTS
PROCEDE DE PRODUCTION DE SELS D'IMIDAZOLIUM A SUBSTITUTION EN POSITION 1,3 PURIFIES

(30) Priorität: 21.07.2003 DE 10333239
(43) Veröffentlichungstag der Anmeldung: 03.05.2006
(73) Patentinhaber: BASF Aktiengesellschaft, 67056 Ludwigshafen (DE)
(72) Erfinder: MAASE, Matthias, 67346 Speyer (DE); MASSONNE, Klemens, 67098 Bad Dürkheim (DE)
(86) Internationale Anmeldenummer: PCT/EP2004/007076
(87) Internationale Veröffentlichungsnummer: WO 2005/019183

(56) Entgegenhaltungen:
- EP-A- 1 182 196
- WO-A-01/77081
- WO-A-03/013685
- ARDUENGO A J ET AL: "ELECTRONIC STABILIZATION OF NUCLEOPHILIC CARBENES" JOURNAL OF THE AMERICAN CHEMICAL SOCIETY, AMERICAN CHEMICAL SOCIETY, WASHINGTON, DC, US, Bd. 114, Nr. 4, 1. Juli 1992 (1992-07-01), Seiten 5530-5534, XP002032799 ISSN: 0002-7863 in der Anmeldung erwähnt

## Beschreibung

Die vorliegende Erfindung betrifft ein Verfahren zur Herstellung von gereinigten 1,3-substituierten Imidazoliumsalzen der allgemeinen Formel (I) 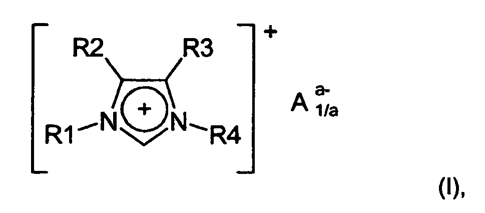 in der
* die Reste R¹, R², R³ und R⁴ unabhängig voneinander einen Kohlenstoff enthaltenden organischen, gesättigten oder ungesättigten, acyclischen oder cyclischen, aliphati-schen, aromatischen oder araliphatischen Rest mit 1 bis 30 Kohlenstoffatomen, welcher ein oder mehrere Heteroatome enthalten und/oder durch eine oder mehrere funktionelle Gruppen oder Halogen substituiert sein kann, bedeuten, wobei die jeweils benachbarten Reste R¹ und R², R² und R³ sowie R³ und R⁴ auch miteinander verbunden sein können und die Reste R² und R³ zusätzlich noch unabhängig voneinander Wasserstoff, Halogen oder eine funktionelle Gruppe bedeuten;
und
* A^{a-} für das teil- oder volldeprotonierte Anion einer anorganischen oder organischen Protonensäure HₐA (III) steht, wobei a eine ganze, positive Zahl ist und den Ladungs-zustand des Anions wiedergibt,
durch Umsetzung eines 1,3-substituierten Imidazoliumsalzes der allgemeinen Formel (II), 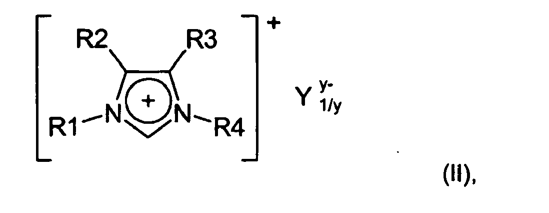 in der die Reste R¹, R², R³ und R⁴ die oben genannte Bedeutung besitzen und das Anion Y^{y-} für das teil- oder volldeprotonierte Anion einer anorganischen oder organischen Protonensäure H_{y}Y (IV) steht, wobei y eine ganze, positive Zahl ist und den Ladungszustand des Anions wiedergibt, mit einer starken Base bei einer Temperatur im Bereich von 20 bis 250°C unter Abdestillation des gebildeten 1,3-substituierten Imidazol-2-ylidens.

1,3-substituierte Imidazoliumsalze gehören zur Gruppe der sogenannten ionischen Flüssigkeiten und gewinnen zunehmend an Bedeutung. So stellen diese beispielsweise für die verschiedensten Anwendungen gute Lösungsmittel für organische und anorganische Verbindungen dar.

Ionische Flüssigkeiten gewinnen als Lösungsmittel wie beispielsweise zur Durchführung chemischer Reaktionen zunehmend an Bedeutung. P. Wasserscheid et al., Angew. Chem. 112 (2000), Seite 3926 bis 3945 gibt etwa einen Überblick über deren Einsatz in der homogenen Übergangsmetallkatalyse. Von großer Bedeutung ist dabei die Reinheit der eingesetzten ionischen Flüssigkeit, da Verunreinigungen den Verlauf der chemischen Reaktionen im Allgemeinen negativ beeinflussen. So weisen beispielsweise P. Dyson et al. in Electrochemical Society Proceedings, Volume 99-41, Seite 161 bis 168 auf Probleme beim Einsatzes von chloridhaltigen ionischen Flüssigkeiten in der Flüssigphasenhydrierung und bei der Suzuki-Reaktion hin. Bei der Herstellung ionischer Flüssigkeiten sind daher hohe Anforderungen an die Reinheit des gewünschten Produkts zu stellen.

Eine wichtige Gruppe der ionischen Flüssigkeiten stellen die substituierten Imidazoliumsalze dar. Im Allgemeinen werden diese aus dem entsprechenden N-substituierten Imidazol durch Alkylierung des zweiten Stickstoffs gewonnen.

WO 02/34722 beschreibt zwei verschiedene Wege zur Herstellung von 1,2,3-substituierten, von 1,2,3,4-substituierten und von 1,2,3,4,5-substituierten Imidazoliumsalzen: (1) Bei der indirekten Route wird das entsprechende, substituierte Imidazol mit einem organischen Halogenid umgesetzt und das Halogenidion des substituierten Imidazolium-halogenids durch Ionenaustausch durch das gewünschte Anion ausgetauscht. (2) Bei der direkten Route wird das entsprechende, substituierte Imidazol mit einem Alkyltriflat oder einem Trialkyloxoniumsalz des gewünschten Anions (z.B. Triethyloxonium-tetrafluoroborat) umgesetzt. Nachteilig an der Herstellung über die indirekte Route (1) ist der hohe Aufwand durch den nachfolgend erforderlichen lonenaustausch und die gegebenenfalls damit verbundene Extraktion des Produkts mit einem organischen Lösungsmittel sowie der verbleibende Restgehalt an Halogenidionen, welcher beispielsweise die Übergangsmetall katalysierten Reaktionen negativ beeinflussen kann. Nachteilig an der Herstellung über die direkte Route (2) ist, dass das Alkylierungsreagenz sowohl den Substituenten als auch das Anion festlegt und somit die Variationsbreite und Flexibiltät in Bezug auf das Anion einschränkt.

EP-A 1 182 196 lehrt die halogenidfreie Herstellung von 1,3-substituierten Imidazoliumsalzen durch Umsetzung des zugrundeliegenden 1-substituierten Imidazols mit dem entsprechenden organischen Disulfat als Alkylierungsagens und anschließendem Ionenaustausch mit einem Metallsalz, welches das gewünschte Anion enthält. So erfolgt beispielsweise die Herstellung von 1-Butyl-3-methyl-imidazolium-tetrafluoroborat durch Umsetzung von 1-Butylimidazol mit Dimethylsulfat, nachfolgender Behandlung mit Natrium-tetrafluoroborat und mehrfache Extraktion des Produkts mit Methylenchlorid. Das genannte Verfahren führt zwar zu anscheinend chloridfreien 1,3-substituierten Imidazoliumsalzen, besitzt aber den entscheidenden Nachteil des Einsatzes von organischem Disulfat, welches aufgrund seiner karzinogenen und ätzenden Wirkung ein erhöhtes Sicherheitsrisiko darstellt und daher einen enormen Sicherheitsaufwand bedingt. Des Weiteren ist der Ionenaustausch durch Zugabe eines Metallsalzes, welches das gewünschte Anion enthält, sehr aufwändig, zumal der Ansatz anschließend extraktiv unter Einsatz eines organischen Lösungsmittels aufzuarbeiten ist.

WO 01/40146 beschreibt die Herstellung von 1,3-substituierten Imidazoliumsalzen durch Umsetzung des zugrundeliegenden 1-substituierten Imidazols mit einem fluorierten Ester, wie beispielsweise einem Trifluoressigsäureester, oder einem Alkylsulfonat als Alkylierungsagens in Gegenwart eines Lösungsmittels. Durch anschließende Umsetzung des erhaltenen Produkts mit der Säure des gewünschten Anions, wie beispielsweise Tetrafluorborsäure oder Hexafluorphosphorsäure, wird das gewünschte 1,3-substituierte Imidazoliumsalz nahezu halogenid- und metallfrei gewonnen. Nachteilig an diesem Verfahren ist die Verunreinigung des gewünschten 1,3-substituierten Imidazoliumsalzes mit Spuren der in der Synthese eingesetzten Verbindungen beziehungsweise deren Reaktionsprodukten, wie beispielsweise von überschüssigem Alkylierungsagens, Salzen des eingesetzten 1-substituierten Imidazols, des eingesetzten Lösungsmittels oder dem 1,3-substituierten Imidazoliumsalz mit dem fluorierten Säureanion oder dem Sulfonat.

WO 96/18459 offenbart die Herstellung halogenidfreier ionischer Flüssigkeiten durch Umsetzung eines Halogenids des gewünschten Imidazolium-, Pyridinium- oder Phosphonium-Kations mit einem Bleisalz, dessen Anion das für die ionische Flüssigkeit gewünschte Anion darstellt und die Abtrennung des ausgefallenen Bleihalogenids. Nachteilig an dem beschriebenen Verfahren ist neben dem Einsatz stöchiometrischer Mengen an toxischen Bleisalzen und der damit verbundene Anfall stöchiometrischer Mengen an zu entsorgenden Bleihalogeniden vor allem die signifikante Verunreinigung des Produkts mit diesen Bleiverbindungen, wie insbesondere aus J.T. Hamill et al., Chem. Comm. 2000, Seite 1929 bis 1930 hervorgeht.

Nachteilig an den oben genannten Verfahren zur Herstellung von substituierten Imidazoliumsalzen ist der Erhalt eines mit Nebenprodukten verunreinigten Produkts, welches bei der Verwendung der substituierten Imidazoliumsalze zu negativen Auswirkungen führen kann.

WO 01/77081 offenbart ein Verfahren, bei dem gereinigte 1,3-substituierte Imidazoliumsalze, welche weder mit Halogenid- noch mit Metallionen verunreinigt sind, hergestellt werden können. Als Einsatzstoff dient ein 1,3-substituiertes Imidazoliumsalz, welches beispielsweise auf herkömmlichem Wege hergestellt wurde, wobei das Anion von dem später gewünschten Anion durchaus verschieden sein kann. Das 1,3-substituierte Imidazoliumsalz wird bei dem genannten Verfahren in Gegenwart einer starken Base, beispielsweise einem Alkoholat, unter reduziertem Druck erhitzt, wobei sich das entsprechende 1,3-substituierte Imidazolium-Carben bildet und abdestilliert. Dieses wird in einer Vorlage als Reinstoff kondensiert. Anschließend wird das kondensierte Carben mit der Säure des gewünschten Anions oder einem Alkohol unter Bildung des gereinigte 1,3-substituierten Imidazoliumsalzes umgesetzt. Nachteilig an diesem Verfahren ist die in A.J. Arduengo et al., J. Am. Chem. Soc. 114, 1992, Seite 5530 bis 5534 beschriebene Tatsache, dass sterisch nicht stark gehinderte Carbene zwar in Lösung für einige Tage stabil sind, jedoch als Reinsubstanz sehr instabil sind. So zersetzt sich beispielsweise reines 1,3-Dimethylimidazol-2-yliden selbst bei tiefen Temperaturen unter Braunfärbung und Bildung eines viskosen Produkts. Selbst wenn durch die in WO 01/77081 genannten Beispiele gezeigt werden konnte, dass 1-Ethyl-3-methylimidazol-2-yliden, 1-Butyl-3-methylimidazol-2-yliden, 1-Hexyl-3-methylimidazol-2-yliden und 1-Octyl-3-methylimidazol-2-yliden in sehr kleinen Mengen in einer Kugelrohr-Apparatur gewonnen und NMR-spektroskopisch nachgewiesen werden konnte, führen die zwangsläufig gebildeten viskosen Nebenprodukte zu einer erneuten Verunreinigung des gewünschten 1,3-substituierten Imidazoliumsalzes. Die Menge der viskosen Nebenprodukte nimmt dabei mit steigender Größe des Produktionsansatzes aufgrund der längeren Verweilzeit noch zu, so dass bei einer technischen Anwendung des Verfahrens nur ein stark verunreinigtes Produkt erhalten werden kann. Ein starkes Herunterkühlen der Destillationsvorlage auf Temperaturen unterhalb 0°C ist technisch sehr aufwändig und energieintensiv.

Demgemäß bestand die Aufgabe, ein Verfahren zur Herstellung von gereinigten 1,3-substituierten Imidazoliumsalzen zu finden, welches die oben genannten Nachteile nicht mehr besitzt, in Bezug auf die Wahl der Substituenten des Imidazolium-Kations und die Wahl des Anions eine große Variabilität und Flexibilität aufweist und in technisch einfacher Art und Weise mit hoher Ausbeute zu reinen bis hochreinen 1,3-substituierten Imidazoliumsalzen führt.

Demgemäß wurde ein Verfahren zur Herstellung von gereinigten 1,3-substituierten Imidazoliumsalzen der allgemeinen Formel (I) 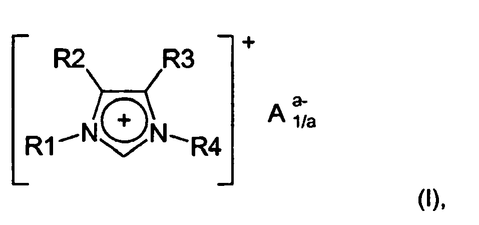 in der
* die Reste R¹, R², R³ und R⁴ unabhängig voneinander einen Kohlenstoff enthaltenden organischen, gesättigten oder ungesättigten, acyclischen oder cyclischen, aliphatischen, aromatischen oder araliphatischen Rest mit 1 bis 30 Kohlenstoffatomen, welcher ein oder mehrere Heteroatome enthalten und/oder durch eine oder mehrere funktionelle Gruppen oder Halogen substituiert sein kann, bedeuten, wobei die jeweils benachbarten Reste R¹ und R², R² und R³ sowie R³ und R⁴ auch miteinander verbunden sein können und die Reste R² und R³ zusätzlich noch unabhängig voneinander Wasserstoff, Halogen oder eine funktionelle Gruppe bedeuten;
und
* A^{a-} für das teil- oder volldeprotonierte Anion einer anorganischen oder organischen Protonensäure HₐA (III) steht, wobei a eine ganze, positive Zahl ist und den Ladungszustand des Anions wiedergibt,
durch Umsetzung eines 1,3-substituierten Imidazoliumsalzes der allgemeinen Formel (II), 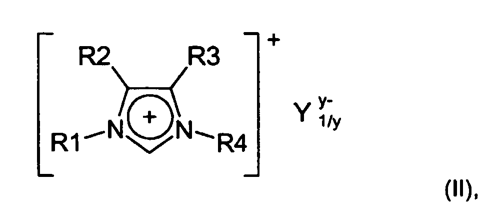 in der die Reste R¹, R², R³ und R⁴ die oben genannte Bedeutung besitzen und das Anion Y^{y-} für das teil- oder volldeprotonierte Anion einer anorganischen oder organischen Protonensäure H_{y}Y (IV) steht, wobei y eine ganze, positive Zahl ist und den Ladungszustand des Anions wiedergibt, mit einer starken Base bei einer Temperatur im Bereich von 20 bis 250°C unter Abdestillation des gebildeten 1,3-substituierten Imidazol-2-ylidens gefunden, welches dadurch gekennzeichnet ist, dass man das abdestillierte 1,3-substituierte Imidazol-2-yliden im gasförmigen Zustand mit der Protonensäure HₐA (III) in Kontakt bringt und/oder das abdestillierte 1,3-substituierte Imidazol-2-yliden im gasförmigen oder kondensierten Zustand in eine, die Protonensäure HₐA (III) enthaltende Vorlage leitet.

Das durch das erfindungsgemäße Verfahren herstellbare gereinigte 1,3-substituierte Imidazoliumsalz (I) enthält das 1,3-substituierte Imidazoliumkation der allgemeinen Formel (Ia) 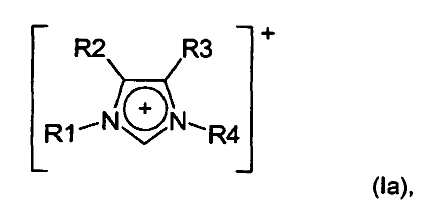 in der
* die Reste R¹, R², R³ und R⁴ unabhängig voneinander einen Kohlenstoff enthaltenden organischen, gesättigten oder ungesättigten, acyclischen oder cyclischen, aliphatischen, aromatischen oder araliphatischen Rest mit 1 bis 30 Kohlenstoffatomen, welcher ein oder mehrere Heteroatome enthalten und/oder durch eine oder mehrere funktionelle Gruppen oder Halogen substituiert sein kann, bedeuten, wobei die jeweils benachbarten Reste R¹ und R², R² und R³ sowie R³ und R⁴ auch miteinander verbunden sein können und die Reste R² und R³ zusätzlich noch unabhängig voneinander Wasserstoff, Halogen oder eine funktionelle Gruppe bedeuten.

Als Heteroatome kommen prinzipiell alle Heteroatome in Frage, welche in der Lage sind, formell eine -CH₂-, eine -CH=, eine C≡ oder eine =C= -Gruppe zu ersetzen. Enthält der Kohlenstoff enthaltende Rest Heteroatome, so sind Sauerstoff, Stickstoff, Schwefel, Phosphor und Silizium bevorzugt. Als bevorzugte Gruppen seien insbesondere -O-, -S-, -SO-, -SO₂-. -NR-, -N=, -PR-, -PR₂ und -SiR₂- genannt, wobei es sich bei den Resten R um den verbleibenden Teil des Kohlenstoff enthaltenden Rests handelt. Der Kohlenstoff enthaltende Rest kann dabei im Falle von R² und R³ auch direkt über das Heteroatom an den Imidazoliumring gebunden sein.

Als funktionelle Gruppen kommen prinzipiell alle funktionellen Gruppen in Frage, welche an ein Kohlenstoffatom oder ein Heteroatom gebunden sein können. Als geeignete Beispiele seien -OH (Hydroxy), =O (insbesondere als Carbonylgruppe), -NH₂ (Amino), =NH (Imino), -COOH (Carboxy), -CONH₂ (Carboxamid) und -CN (Cyano) genannt. Fuktionelle Gruppen und Heteroatome können auch direkt benachbart sein, so dass auch Kombinationen aus mehreren benachbarten Atomen, wie etwa -O- (Ether), -S-(Thioether), -COO- (Ester), -CONH- (sekundäres Amid) oder -CONR- (tertiäres Amid), mit umfasst sind, beispielsweise Di-(C₁-C₄-Alkyl)-amino, C₁-C₄-Alkyloxycarbonyl oder C₁-C₄-Alkyloxy.

Als Halogene seien Fluor, Chlor, Brom und Iod genannt.

Bevorzugt werden beim erfindungsgemäßen Verfahren gereinigte 1,3-substituierte Imidazoliumsalze (I) hergestellt, bei denen die Reste R² und R³ unabhängig voneinander
- Wasserstoff;
- Halogen; oder
- eine funktionelle Gruppe;
und die Reste R¹, R², R³ und R⁴ unabhängig voneinander jeweils
• gegebenenfalls durch funktionelle Gruppen, Aryl, Alkyl, Aryloxy, Alkyloxy, Halogen, Heteroatome und/oder Heterocyclen substituiertes und/oder durch ein oder mehrere Sauerstoff- und/oder Schwefelatome und/oder ein oder mehrere substituierte oder unsubstituierte Iminogruppen unterbrochenes C₁- bis C₁₈-Alkyl;
• gegebenenfalls durch funktionelle Gruppen, Aryl, Alkyl, Aryloxy, Alkyloxy, Halogen, Heteroatome und/oder Heterocyclen substituiertes und/oder durch ein oder mehrere Sauerstoff- und/oder Schwefelatome und/oder ein oder mehrere substituierte oder unsubstituierte Iminogruppen unterbrochenes C₂- bis C₁₈-Alkenyl;
• gegebenenfalls durch funktionelle Gruppen, Aryl, Alkyl, Aryloxy, Alkyloxy, Halogen, Heteroatome und/oder Heterocyclen substituiertes C₆- bis C₁₂-Aryl;
• gegebenenfalls durch funktionelle Gruppen, Aryl, Alkyl, Aryloxy, Alkyloxy, Halogen, Heteroatome und/oder Heterocyclen substituiertes C₅- bis C₁₂-Cycloalkyl;
• gegebenenfalls durch funktionelle Gruppen, Aryl, Alkyl, Aryloxy, Alkyloxy, Halogen, Heteroatome und/oder Heterocyclen substituiertes C₅- bis C₁₂-Cycloalkenyl; oder
• einen gegebenenfalls durch funktionelle Gruppen, Aryl, Alkyl, Aryloxy, Alkyloxy, Halogen, Heteroatome und/oder Heterocyclen substituierten fünf- bis sechsgliedrigen, Sauerstoff-, Stickstoff- und/oder Schwefelatome aufweisenden Heterocyclus bedeuten; oder
benachbarte Reste R¹ und R², R² und R³ sowie R³ und R⁴ gemeinsam
• einen ungesättigten, gesättigten oder aromatischen, gegebenenfalls durch funktionelle Gruppen, Aryl, Alkyl, Aryloxy, Alkyloxy, Halogen, Heteroatome und/oder Heterocyclen substituierten und gegebenenfalls durch ein oder mehrere Sauerstoff- und/oder Schwefelatome und/oder ein oder mehrere substituierte oder unsubstituierte Iminogruppen unterbrochenen Ring bilden.

Bei gegebenenfalls durch funktionelle Gruppen, Aryl, Alkyl, Aryloxy, Alkyloxy, Halogen, Heteroatome und/oder Heterocyclen substituiertem C₁- bis C₁₈-Alkyl handelt es sich bevorzugt um Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, Heptyl, Octyl, 2-Etylhexyl, 2,4,4-Trimethylpentyl, 1,1,3,3-Tetramethylbutyl, 1-Nonyl, 1-Decyl, 1-Undecyl, 1-Dodecyl, 1-Tridecyl, 1-Tetradecyl, 1-Pentadecyl, 1-Hexadecyl, 1-Heptadecyl, 1-Octadecyl, Cyclopentylmethyl, 2-Cyclopentylethyl, 3-Cyclopentylpropyl, Cyclohexylmethyl, 2-Cyclohexylethyl, 3-Cyclohexylpropyl, Benzyl (Phenylmethyl), Diphenylmethyl (Benzhydryl), Triphenylmethyl, 1-Phenylethyl, 2-Phenylethyl, 3-Phenylpropyl, α,α-Dimethylbenzyl, p-Tolylmethyl, 1-(p-Butylphenyl)-ethyl, p-Chlorbenzyl, 2,4-Dichlorbenzyl, p-Methoxybenzyl, m-Ethoxybenzyl, 2-Cyanoethyl, 2-Cyanopropyl, 2-Methoxycarbonethyl, 2-Ethoxycarbonylethyl, 2-Butoxycarbonylpropyl, 1,2-Di-(methoxycarbonyl)-ethyl, Methoxy, Ethoxy, Formyl, 1,3-Dioxolan-2-yl, 1,3-Dioxan-2-yl, 2-Methyl-1,3-dioxotan-2-yl, 4-Methyl-1,3-dioxolan-2-yl, Chlormethyl, 2-Chlorethyl, Trichlormethyl, Trifluormethyl, 1,1-Dimethyl-2-chlorethyl, 2,2,2-Trifluorethyl, 2-Hydroxyethyl, 2-Hydroxypropyl, 3-Hydroxypropyl, 4-Hydroxybutyl, 6-Hydroxyhexyl, 2-Aminoethyl, 2-Aminopropyl, 3-Aminopropyl, 4-Aminobutyl, 6-Aminohexyl, 2-Methylaminoethyl, 2-Methylaminopropyl, 3-Methylaminopropyl, 4-Methylaminobutyl, 6-Methylaminohexyl, 2-Dimethylaminoethyl, 2-Dimethylaminopropyl, 3-Dimethylaminopropyl, 4-Dimethylaminobutyl, 6-Dimethylaminohexyl, 2-Hydroxy-2,2-dimethylethyl, 2-Phenoxyethyl, 2-Phenoxypropyl, 3-Phenoxypropyl, 4-Phenoxybutyl, 6-Phenoxyhexyl, 2-Methoxyethyl, 2-Methoxypropyl, 3-Methoxypropyl, 4-Methoxybutyl, 6-Methoxyhexyl, 2-Ethoxyethyl, 2-Ethoxypropyl, 3-Ethoxypropyl, 4-Ethoxybutyl, 6-Ethoxyhexyl, Acetyl, CₙF_{2(n-a)+(1-b)}H_{2a+b} mit n ≤ 30, 0 ≤ a ≤ n und b = 0 oder 1 (beispielsweise CF₃, C₂F₅, CH₂CH₂-C₍ₙ₋₂₎F₂₍ₙ₋₂₎₊₁, C₆F₁₃, C₈F₁₇, C₁₀F₂₁, C₁₂F₂₅). Methoxymethyl, 2-Methoxyethyl, 2-Ethoxyethyl, 2-Butoxyethyl, Diethoxymethyl, Diethoxyethyl, 2-Isopropoxyethyl, 2-Butoxypropyl, 2-Octyloxyethyl, 2-Methoxyisopropyl, 2-(Methoxycarbonyl)-ethyl, 2-(Ethoxycarbonyl)-ethyl, 2-(n-Butoxycarbonyl)-ethyl, Butylthiomethyl, 2-Dodecylthioethyl, 2-Phenylthioethyl, 5-Hydroxy-3-oxa-pentyl, 8-Hydroxy-3,6-dioxa-octyl, 11-Hydroxy-3,6,9-trioxa-undecyl, 7-Hydroxy-4-oxa-heptyl, 11-Hydroxy-4,8-dioxa-undecyl, 15-Hydroxy-4,8,12-trioxa-pentadecyl, 9-Hydroxy-5-oxa-nonyl, 14-Hydroxy-5,10-oxa-tetradecyl, 5-Methoxy-3-oxa-pentyl, 8-Methoxy-3,6-dioxa-octyl, 11-Methoxy-3,6,9-trioxa-undecyl, 7-Methoxy-4-oxa-heptyl, 11-Methoxy-4,8-dioxa-undecyl, 15-Methoxy-4,8,12-trioxa-pentadecyl, 9-Methoxy-5-oxa-nonyl, 14-Methoxy-5,10-oxa-tetradecyl, 5-Ethoxy-3-oxa-pentyl, 8-Ethoxy-3,6-dioxa-octyl, 11-Ethoxy-3,6,9-trioxa-undecyl, 7-Ethoxy-4-oxa-heptyl, 11-Ethoxy-4,8-dioxa-undecyl, 15-Ethoxy-4,8,12-trioxa-pentadecyl, 9-Ethoxy-5-oxa-nonyl oder 14-Ethoxy-5,10-oxa-tetradecyl.

Bei gegebenenfalls durch funktionelle Gruppen, Aryl, Alkyl, Aryloxy, Alkyloxy, Halogen, Heteroatome und/oder Heterocyclen substituiertes und/oder durch ein oder mehrere Sauerstoff- und/oder Schwefelatome und/oder ein oder mehrere substituierte oder unsubstituierte Iminogruppen unterbrochenes C₂- bis C₁₈-Alkenyl handelt es sich bevorzugt um Vinyl, 2-Propenyl, 3-Butenyl, cis-2-Butenyl, trans-2-Butenyl oder CₙF_{2(n-a)-(1-b)}H_{2a-b} mit n ≤30, 0 ≤ a ≤ n und b = 0 oder 1.

Bei gegebenenfalls durch funktionelle Gruppen, Aryl, Alkyl, Aryloxy, Alkyloxy, Halogen, Heteroatome und/oder Heterocyclen substituiertes C₆- bis C₁₂-Aryl handelt es sich bevorzugt um Phenyl, Tolyl, Xylyl, α-Naphthyl, β-Naphthyl, 4-Diphenylyl, Chlorphenyl, Dichlorphenyl, Trichlorphenyl, Difluorphenyl, Methylphenyl, Dimethylphenyl, Trimethylphenyl, Ethylphenyl, Diethylphenyl, iso-Propylphenyl, tert.-Butylphenyl, Dodecylphenyl, Methoxyphenyl, Dimethoxyphenyl, Ethoxyphenyl, Hexyloxyphenyl, Methylnaphthyl, Isopropylnaphthyl, Chlomaphthyl, Ethoxynaphthyl, 2,6-Dimethylphenyl, 2,4,6-Trimethylphenyl, 2,6-Dimethoxyphenyl, 2,6-Dichlorphenyl, 4-Bromphenyl, 2-Nitrophenyl, 4-Nitrophenyl, 2,4-Dinitrophenyl, 2,6-Dinitrophenyl, 4-Dimethylaminophenyl, 4-Acetylphenyl, Methoxyethylphenyl, Ethoxymethylphenyl, Methylthiophenyl, Isopropylthiophenyl oder tert.-Butylthiophenyl oder C₆F₍₅₋ₐ₎Hₐ mit 0 ≤ a ≤ 5.

Bei gegebenenfalls durch funktionelle Gruppen, Aryl, Alkyl, Aryloxy, Alkyloxy, Halogen, Heteroatome und/oder Heterocyclen substituiertes C₅- bis C₁₂-Cycloalkyl handelt es sich bevorzugt um Cyclopentyl, Cyclohexyl, Cyclooctyl, Cyclododecyl, Methylcyclopentyl, Dimethylcyclopentyl, Methylcyclohexyl, Dimethylcyclohexyl, Diethylcyclohexyl, Butylcyclohexyl, Methoxycyclohexyl, Dimethoxycyclohexyl, Diethoxycyclohexyl, Butylthiocyclohexyl, Chlorcyclohexyl, Dichlorcyclohexyl, Dichlorcyclopentyl, CₙF_{2(n-a)-(1-b)}H_{2a-b} mit n ≤ 30, 0 ≤ a ≤ n und b = 0 oder 1 sowie ein gesättigtes oder ungesättigtes bicyclisches System wie z.B. Norbomyl oder Norbornenyl.

Bei gegebenenfalls durch funktionelle Gruppen, Aryl, Alkyl, Aryloxy, Alkyloxy, Halogen, Heteroatome und/oder Heterocyclen substituiertes C₅- bis C₁₂-Cycloalkenyl handelt es sich bevorzugt um 3-Cyclopentenyl, 2-Cyclohexenyl, 3-Cyclohexenyl, 2,5-Cyclohexadienyl oder CₙF_{2(n-a)-3(1-b)}H_{2a-3b} mit n ≤ 30, 0 ≤ a ≤ n und b = 0 oder 1.

Bei einen gegebenenfalls durch funktionelle Gruppen, Aryl, Alkyl, Aryloxy, Alkyloxy, Halogen, Heteroatome und/oder Heterocyclen substituierten fünf- bis sechsgliedrigen, Sauerstoff-, Stickstoff- und/oder Schwefelatome aufweisenden Heterocyclus handelt es sich bevorzugt um Furyl, Thiophenyl, Pyrryl, Pyridyl, Indolyl, Benzoxazolyl, Dioxolyl, Dioxyl, Benzimidazolyl, Benzthiazolyl, Dimethylpyridyl, Methylchinolyl, Dimethylpyrryl, Methoxyfuryl, Dimethoxypyridyl oder Difluorpyridyl.

Bilden die benachbarte Reste R¹ und R², R² und R³ sowie R³ und R⁴ gemeinsam einen ungesättigten, gesättigten oder aromatischen, gegebenenfalls durch funktionelle Gruppen, Aryl, Alkyl, Aryloxy, Alkyloxy, Halogen, Heteroatome und/oder Heterocyclen substituierten und gegebenenfalls durch ein oder mehrere Sauerstoff und/oder Schwefelatome und/oder ein oder mehrere substituierte oder unsubstituierte Iminogruppen unterbrochenen Ring, so handelt es sich bevorzugt um 1,3-Propylen, 1,4-Butylen, 2-Oxa-1,3-propylen, 1-Oxa-1,3-propylen, 2-Oxa-1,3-propylen, 1-Oxa-1,3-propenylen, 1-Aza-1,3-propenylen, 1-C₁-C₄-Alkyl-1-aza-1,3-propenylen, 1,4-Buta-1,3-dienylen, 1-Aza-1,4-buta-1,3-dienylen oder 2-Aza-1,4-buta-1,3-dienylen.

Enthalten die oben genannten Reste Sauerstoff- und/oder Schwefelatome und/oder substituierte oder unsubstituierte Iminogruppen, so ist die Anzahl der Sauerstoff- und/oder Schwefelatome und/oder Iminogruppen nicht beschränkt. In der Regel beträgt sie nicht mehr als 5 in dem Rest, bevorzugt nicht mehr als 4 und ganz besonders bevorzugt nicht mehr als 3.

Enthalten die oben genannten Reste Heteroatome, so befinden sich zwischen zwei Heteroatomen in der Regel mindestens ein Kohlenstoffatom, bevorzugt mindestens zwei Kohlenstoffatome.

Besonders bevorzugt stehen die Reste R¹ und R⁴ unabhängig voneinander für unverzweigtes oder verzweigtes C₁- bis C₁₂-Alkyl, wie beispielsweise Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, 1-Heptyl, 1-Octyl, 1-Nonyl, 1-Decyl, 1-Undecyl, 1-Dodecyl, 1-Tetradecyl, 1-Hexadecyl, 1-Octadecyl, 2-Hydroxyethyl, Benzyl, 3-Phenylpropyl, Vinyl, 2-Cyanoethyl, 2-(Methoxycarbonyl)-ethyl, 2-(Ethoxycarbonyl)-ethyl, 2-(n-Butoxycarbonyl)-ethyl, Dimethylamino, Diethylamino, Trifluormethyl, Difluormethyl, Fluormethyl, Pentafluorethyl, Heptafluorpropyl, Heptafluorisopropyl, Nonafluorbutyl, Nonafluorisobutyl, Undecylfluorpentyl, Undecylfluorisopentyl, für 6-Hydroxyhexyl oder Propylsulfonsäure.

Besonders bevorzugt stehen die Reste R² und R³ unabhängig voneinander für Wasserstoff oder unverzweigtes oder verzweigtes C₁- bis C₁₂-Alkyl, wie beispielsweise Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, Heptyl, Octyl, Nonyl, Decyl, Undecyl, Dodecyl, 2-Hydroxyethyl, 2-Cyanoethyl, 2-(Methoxycarbonyl)-ethyl, 2-(Ethoxycarbonyl)-ethyl, 2-(n-Butoxycarbonyl)-ethyl, Dimethylamino, Diethylamino, Chlor, Trifluormethyl, Difluormethyl, Fluormethyl, Pentafluorethyl, Heptafluorpropyl, Heptafluorisopropyl, Nonafluorbutyl, Nonafluorisobutyl, Undecylfluorpentyl, Undecylfluorisopentyl oder für 6-Hydroxyhexyl.

Ganz besonders bevorzugt stellt man beim erfindungsgemäßen Verfahren ein gereinigtes 1,3-substituiertes Imidazoliumsalz (1) her, bei dem die Reste R¹ und R⁴ unabhängig voneinander für Methyl, Ethyl, 1-Propyl, 2-Propyl, 1-Butyl, 1-Pentyl, 1-Hexyl, 1-Heptyl, 1-Octyl, 1-Nonyl, 1-Decyl, 1-Dodecyl, 1-Tetradecyl, 1-Hexadecyl, 1-Octadecyl, 1-(2-Ethyl)hexyl, Benzyl, 3-Phenylpropyl, 6-Hydroxyhexyl oder Phenyl und die Reste R² und R³ unabhängig voneinander für Wasserstoff, Methyl, Ethyl, n-Propyl, 2-Propyl, 1,-Butyl, 1-Hexyl, 6-Hydroxyhexyl, Phenyl oder Chlor stehen.

Ganz besonders bevorzugt stellt man beim erfindungsgemäßen Verfahren ein gereinigtes 1,3-substituiertes Imidazoliumsalz (I) her, welches als 1,3-substituiertes Imidazoliumkation 1,3-Dimethylimdazolium, 1-Ethyl-3-methylimidazolium, 1-Methyl-3-propyl-imidazolium, 1-Isopropyl-3-methylimidazolium, 1-Butyl-3-methylimidazolium, 1-Methyl-3-pentylimidazolium, 1-Hexyl-3-methylimidazolium, 1-Heptyl-3-methylimidazolium, 1-Methyl-3-octylimidazolium, 1-Decyl-3-methylimidazolium, 1-Methyl-3-benzylimidazolium, 1-Methyl-3-(3-phenylpropyl)imidazolium, 1-(2-Ethyl)hexyl-3-methylimidazolium, 1-Methyl-3-nonylimidazolium, 1-Methyl-3-decylimidazolium, 1,2,3-Trimethylimidazolium, 1-Ethyl-2,3-dimethylimidazolium oder 1-Butyl-2,3-dimethylimidazolium enthält.

Das durch das erfindungsgemäße Verfahren herstellbare gereinigte 1,3-substituierte Imidazoliumsalz (I) enthält das Anion A^{a-}, wobei dieses für das teil- oder volldeprotonierte Anion einer anorganischen oder organischen Protonensäure HₐA (III) steht, wobei a eine ganze, positive Zahl ist und den Ladungszustand des Anions wiedergibt.

Als teildeprotoniertes Anion ist ein Anion aus einer mehrwertigen Säure zu verstehen, welches noch ein oder mehrere deprotonierbare Wasserstoffatome enthält. Entsprechend ist als volldeprotoniertes Anion ein Anion zu verstehen, welches keine weiteren deprotonierbaren Wasserstoffatome enthält.

Bevorzugt werden beim erfindungsgemäßen Verfahren gereinigte 1,3-substituierte Imidazoliumsalze (I) hergestellt, bei denen das Anion A^{a-} für
Fluorid; Hexafluorophosphat; Hexafluoroarsenat; Hexafluoroantimonat; Trifluoroarsenat; Nitrit; Nitrat; Sulfat; Hydrogensulfat; Carbonat; Hydrogencarbonat; Phosphat; Hydrogenphosphat; Dihydrogenphosphat; Vinylphosphonat; Dicyanamid; Bis(pentafluoroethyl)phosphinat; Tris(pentafluoroethyl)trifluorophosphat; Tris(heptafluoropropyl)trifluorophosphat; Bis[oxalato(2-)]borat; Bis[salicylato(2-)]borat; Bis[1,2-benzoldiolato(2-)-O,O']borat; Tetracyanoborat; Tetracarbonylcobaltat;
tetrasubstituiertes Borat der allgemeinen Formel (Va) [BR^{a}R^{b}R^{c}R^{d}]⁻, wobei R^{a} bis R^{d} unabhängig voneinander für Fluor oder einen Kohlenstoff enthaltenden organischen, gesättigten oder ungesättigten, acyclischen oder cyclischen, aliphatischen, aromatischen oder araliphatischen Rest mit 1 bis 30 Kohlenstoffatomen, welcher ein oder mehrere Heteroatome enthalten und/oder durch eine oder mehrere funktionelle Gruppen oder Halogen substituiert sein kann, stehen;
organisches Sulfonat der allgemeinen Formel (Vb) [R^{e}-SO₃]⁻, wobei R^{e} für einen Kohlenstoff enthaltenden organischen, gesättigten oder ungesättigten, acyclischen oder cyclischen, aliphatischen, aromatischen oder araliphatischen Rest mit 1 bis 30 Kohlenstoffatomen, welcher ein oder mehrere Heteroatome enthalten und/oder durch eine oder mehrere funktionelle Gruppen oder Halogen substituiert sein kann, steht;
Carboxylat der allgemeinen Formel (Vc) [R^{f}-COO]⁻, wobei R^{f} für Wasserstoff oder einen Kohlenstoff enthaltenden organischen, gesättigten oder ungesättigten, acyclischen oder cyclischen, aliphatischen, aromatischen oder araliphatischen Rest mit 1 bis 30 Kohlenstoffatomen, welcher ein oder mehrere Heteroatome enthalten und/oder durch eine oder mehrere funktionelle Gruppen oder Halogen substituiert sein kann, steht;
(Fluoralkyl)fluorphosphat der allgemeinen Formel (Vd) [PFₓ(C_{y}F_{2y+1-z}H_{z})₆₋ₓ]⁻, wobei 1 ≤ x ≤ 6, 1 ≤ y ≤ 8 und 0 ≤ z ≤ 2y+1;
Imid der allgemeinen Formeln (Ve) [R^{g}-SO₂-N-SO₂-R^{h}]⁻, (Vf) [Rⁱ-SO₂-N-CO-R^{j}]⁻ oder (Vg) [R^{k}-CO-N-CO-R^{l}]⁻, wobei R^{g} bis R^{l} unabhängig voneinander für Wasserstoff oder einen Kohlenstoff enthaltenden organischen, gesättigten oder ungesättigten, acyclischen oder cyclischen, aliphatischen, aromatischen oder araliphatischen Rest mit 1 bis 30 Kohlenstoffatomen, welcher ein oder mehrere Heteroatome enthalten und/oder durch eine oder mehrere funktionelle Gruppen oder Halogen substituiert sein kann, stehen;
Methid der allgemeinen Formel (Vh) 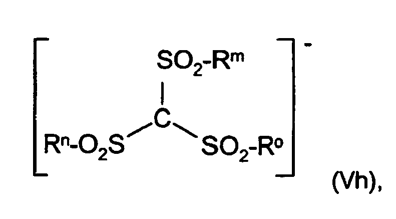 wobei R^{m} bis R^{o} unabhängig voneinander für Wasserstoff oder einen Kohlenstoff enthaltenden organischen, gesättigten oder ungesättigten, acyclischen oder cyclischen, aliphatischen, aromatischen oder araliphatischen Rest mit 1 bis 30 Kohlenstoffatomen, welcher ein oder mehrere Heteroatome enthalten und/oder durch eine oder mehrere funktionelle Gruppen oder Halogen substituiert sein kann, stehen;
organisches Sulfat der allgemeinen Formel (Vi) [R^{p}O-SO₃]⁻, wobei R^{p} für einen Kohlenstoff enthaltenden organischen, gesättigten oder ungesättigten, acyclischen oder cyclischen, aliphatischen, aromatischen oder araliphatischen Rest mit 1 bis 30 Kohlenstoffatomen, welcher ein oder mehrere Heteroatome enthalten und/oder durch eine oder mehrere funktionelle Gruppen oder Halogen substituiert sein kann, steht; oder
Halometallat der allgemeinen Formel (Vj) [M_{q}Halᵣ]^{s-}, wobei M für ein Metall und Hal für Fluor, Chlor, Brom oder Iod steht, q und r ganze positive Zahlen sind und die Stöchiometrie des Komplexes angeben und s eine ganze positive Zahl ist und die Ladung des Komplexes angibt;
steht.

Die Ladung "a-" des Anions A^{a}- beträgt "1-". "2-" oder "3-". Als Beispiele zweifach negativ geladener Anionen seien Sulfat, Hydrogenphosphat und Carbonat genannt. Als Beispiel eines dreifach negativ geladenen Anions sei Phosphat genannt.

Als Heteroatome kommen prinzipiell alle Heteroatome in Frage, welche in der Lage sind, formell eine -CH₂-, eine -CH=, eine C≡ oder eine =C= -Gruppe zu ersetzen. Enthält der Kohlenstoff enthaltende Rest Heteroatome, so sind Sauerstoff, Stickstoff, Schwefel, Phosphor und Silizium bevorzugt. Als bevorzugte Gruppen seien insbesondere -O-, -S-, -SO-, -SO₂-, -NR-, -N=, -PR-, -PR₂ und -SiR₂- genannt, wobei es sich bei den Resten R um den verbleibenden Teil des Kohlenstoff enthaltenden Rests handelt.

Als funktionelle Gruppen kommen prinzipiell alle funktionellen Gruppen in Frage, welche an ein Kohlenstoffatom oder ein Heteroatom gebunden sein können. Als geeignete Beispiele seien -OH (Hydroxy), =O (insbesondere als Carbonylgruppe), -NH₂ (Amino), =NH (Imino), -COOH (Carboxy), -CONH₂ (Carboxamid) und -CN (Cyano) genannt. Fuktionelle Gruppen und Heteroatome können auch direkt benachbart sein, so dass auch Kombinationen aus mehreren benachbarten Atomen, wie etwa -O- (Ether), -S- (Thioether), -COO- (Ester), -CONH- (sekundäres Amid) oder -CONR- (tertiäres Amid), mit umfasst sind,

Als Halogene seien Fluor, Chlor, Brom und Iod genannt.

Als Kohlenstoff enthaltende organische, gesättigte oder ungesättigte, acyclische oder cyclische, aliphatische, aromatische oder araliphatische Reste mit 1 bis 30 Kohlenstoffatomen stehen die Reste R^{a} bis R^{d} beim tetrasubstituiertes Borat (Va), der Rest R^{e} beim organischen Sulfonat (Vb), der Rest R^{f} beim Carboxylat (Vc) und die Reste R^{g} bis R^{l} bei den Imiden (Ve), (Vf) und (Vg) unabhängig voneinander bevorzugt für
- C₁- bis C₃₀-Alkyl und deren aryl-, heteroaryl-, cycloalkyl-, halogen-, hydroxy-, amino-, carboxy-, formyl-, -O-, -CO-, -CO-O- oder -CO-N< substituierte Komponenten, wie beispielsweise Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Icosyl, Henicosyl, Docosyl, Tricosyl, Tetracosyl, Pentacosyl, Hexacosyl, Heptacosyl, Octacosyl, Nonacosyl, Triacontyl, Phenyl methyl (Benzyl), Diphenylmethyl, Triphenylmethyl, 2-Phenylethyl, 3-Phenylpropyl, Cyclopentylmethyl, 2-Cyclopentylethyl, 3-Cyclopentylpropyl, Cyclohexylmethyl, 2-Cyclohexylethyl, 3-Cyclohexylpropyl, Methoxy, Ethoxy, Formyl, Acetyl oder CₙF_{2(n-1)+(1-b)}H_{2a+b} mit n ≤ 30, 0 ≤ a ≤ n und b = 0 oder 1 (beispielsweise CF₃, C₂F₅, CH₂CH₂-C₍ₙ₋₂)F_{2(n-2)+1,} C₆F₁₃, C₈F₁₇, C₁₀F_{21,} C₁₂F₂₅);
- C₃- bis C₁₂-Cycloalkyl und deren aryl-, heteroaryl-, cycloalkyl-, halogen-, hydroxy-, amino-, carboxy-, formyl-, -O-, -CO- oder -CO-O-substituierte Komponenten, wie beispielsweise Cyclopentyl, 2-Methyl-1-cyclopentyl, 3-Methyl-1-cyclopentyl, Cyclohexyl, 2-Methyl-1-cyclohexyl, 3-Methyl-1-cyclohexyl, 4-Methyl-1-cyclohexyl oder CₙF_{2(n-a)-(1-b)}H_{2a-b} mit n ≤ 30, 0 ≤ a ≤ n und b = 0 oder 1;
- C₂- bis C₃₀-Alkenyl und deren aryl-, heteroaryl-, cycloalkyl-, halogen-, hydroxy-, amino-, carboxy-, formyl-, -O-, -CO- oder -CO-O-substituierte Komponenten, wie beispielsweise 2-Propenyl, 3-Butenyl, cis-2-Butenyl, trans-2-Butenyl oder CₙF_{2(n-a)-(1-b)}H_{2a-b} mit n ≤ 30, 0 ≤ a≤ n und b = 0 oder 1;
- C₃- bis C₁₂-Cycloalkenyl und deren aryl-, heteroaryl-, cycloalkyl-, halogen-, hydroxy-, amino-, carboxy-, formyl-, -O-, -CO- oder -CO-O-substituierte Komponenten, wie beispielsweise 3-Cyclopentenyl, 2-Cyclohexenyl, 3-Cyclohexenyl, 2,5-Cyclohexadienyl oder CₙF_{2(n-a)-3(1-b)}H_{2a-3b} mit n ≤ 30, 0 ≤ a ≤ n und b = 0 oder 1; und
- Aryl oder Heteroaryl mit 2 bis 30 Kohlenstoffatomen und deren alkyl-, aryl-, heteroaryl-, cycloalkyl-, halogen-, hydroxy-, amino-, carboxy-, formyl-, -O-, -CO- oder -CO-O-substituierte Komponenten, wie beispielsweise Phenyl, 2-Methyl-phenyl-(2-Tolyl), 3-Methyl-phenyl (3-Tolyl), 4-Methyl-phenyl, 2-Ethyl-phenyl, 3-Ethyl-phenyl, 4-Ethyl-phenyl, 2,3-Dimethyl-phenyl, 2,4-Dimethyl-phenyl, 2,5-Dimethyl-phenyl, 2,6-Dimethyl-phenyl, 3,4-Dimethyl-phenyl, 3,5-Dimethyl-phenyl, 4-Phenylphenyl, 1-Naphthyl, 2-Naphthyl, 1-Pyrrolyl, 2-Pyrrolyl, 3-Pyrrolyl, 2-Pyridinyl, 3-Pyridinyl, 4-Pyridinyl oder C₆F₍₅₋ₐ₎Hₐ mit 0 ≤ a ≤ 5.

Handelt es sich bei dem Anion A^{a-} um ein tetrasubstituiertes Borat (Va) [BR^{a}R^{b}R^{c}R^{d}]⁻, so sind bei diesem bevorzugt alle vier Reste R^{a} bis R^{d} identisch, wobei diese bevorzugt für Fluor, Trifluormethyl, Pentafluorethyl, Phenyl, 3,5-Bis(trifluormethyl)phenyl stehen. Besonders bevorzugte tetrasubstituierte Borate (Va) sind Tetrafluoroborat, Tetraphenylborat und Tetra[3,5-bis(trifluormethyl)phenyl]borat.

Handelt es sich bei dem Anion A^{a-} um ein organisches Sulfonat (Vb) [R^{e}-SO₃]⁻, so steht der Rest R^{e} bevorzugt für Methyl, Trifluormethyl, Pentafluorethyl, p-Tolyl oder C₉F₁₉. Besonders bevorzugte organische Sulfonate (Vb) sind Trifluormethansulfonat (Triflat), Methansulfonat, p-Tolylsulfonat, Nonadecafluorononansulfonat (Nonaflat), Dimethylenglykolmonomethyl-ethersulfat und Octylsulfat.

Handelt es sich bei dem Anion A^{a-} um ein Carboxylat (Vc) [R^{f}-COO]⁻, so steht der Rest R^{f} bevorzugt für Wasserstoff, Trifluormethyl, Pentafluorethyl, Phenyl, Hydroxy-phenylmethyl, Trichlormethyl, Dichlormethyl, Chlormethyl, Trifluormethyl, Difluormethyl, Fluormethyl, Ethenyl (Vinyl), 2-Propenyl, -CH=CH-COO⁻, cis-8-Heptadecenyl, -CH₂-C(OH)(COOH)-CH₂-COO⁻ oder unverzweigtes oder verzweigtes C₁- bis C₁₈-Alkyl, wie beispielsweise Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, Heptyl, Octyl, Nonyl, Decyl, Undecyl, Dodecyl, Heptadecyl. Besonders bevorzugte Carboxylate (Vc) sind Formiat, Acetat, Propionat, Butyrat, Valeriat, Benzoat, Mandelat, Trichloracetat, Dichloracetat, Chloracetat, Trifluoracetat, Difluoracetat, Fluoracetat.

Handelt es sich bei dem Anion A^{a-} um ein (Fluoralkyl)fluorphosphat (Vd) [PFₓ(C_{y}F_{2y+1-z}H_{z})₆₋ₓ]⁻, so ist z bevorzugt 0. Besonders bevorzugt sind (Fluoralkyl)-fluorphosphate (Vd), bei denen z = 0, x = 3 und 1 ≤ y ≤ 4, konkret [PF₃(CF₃)₃]⁻, [PF₃(C₂F₅)₃]⁻, [PF₃(C₃F₇)₃]⁻ und [PF₃(C₄F₇)₃]⁻.

Handelt es sich bei dem Anion A^{a-} um ein Imid (Ve) (R^{g}-SO₂-N-SO₂-R^{h}]⁻, (Vf) [Rⁱ-SO₂-N-CO-R^{j}]- oder (Vg) [R^{k}-CO-N-CO-R^{l}]⁻, so stehen die Reste R^{g} bis R^{l} unabhängig voneinander bevorzugt für Trifluormethyl, Pentafluorethyl, Phenyl, Trichlormethyl, Dichlormethyl, Chlormethyl, Trifluormethyl, Difluormethyl, Fluormethyl oder unverzweigtes oder verzweigtes C₁- bis C₁₂-Alkyl, wie beispielsweise Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, Heptyl, Octyl, Nonyl, Decyl, Undecyl oder Dodecyl. Besonders bevorzugte Imide (Ve), (Vf) und (Vg) sind [F₃C-SO₂-N-SO₂-CF₃]⁻ (Bis(trifluoromethylsulfonyl)imid), [F₅C₂-SO₂-N-SO₂-C₂F₅]⁻ (Bis(pentafluoroethylsulfonyl)imid), [F₃C-SO₂-N-CO-CF₃]⁻, [F₃C-CO-N-CO-CF₃]⁻ und jene, in denen die Reste R^{g} bis R^{l} unabhängig voneinander für Methyl, Ethyl, Propyl, Butyl, Phenyl, Trichlormethyl, Dichlormethyl, Chlormethyl, Trifluormethyl, Difluormethyl oder Fluormethyl stehen.

Handelt es sich bei dem Anion A^{a-} um ein Methid (Vh) 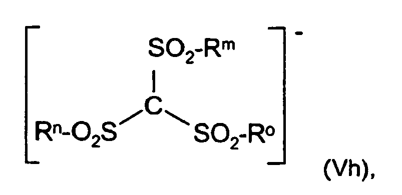 so stehen die Reste R^{m} bis R^{o} unabhängig voneinander bevorzugt für Trifluormethyl, Pentafluorethyl, Phenyl, Trichlormethyl, Dichlormethyl, Chlormethyl, Trifluormethyl, Difluormethyl, Fluormethyl oder unverzweigtes oder verzweigtes C₁- bis C₁₂-Alkyl, wie beispielsweise Methyl, Ethyl, 1-Propyl, 2-Propyl, 1-Butyl, 2-Butyl, 2-Methyl-1-propyl (Isobutyl), 2-Methyl-2-propyl (tert.-Butyl), 1-Pentyl, 2-Pentyl, 3-Pentyl, 2-Methyl-1-butyl, 3-Methyl-1-butyl, 2-Methyl-2-butyl, 3-Methyl-2-butyl, 2,2-Dimethyl-1-propyl, 1-Hexyl, 2-Hexyl, 3-Hexyl, 2-Methyl-1-pentyl, 3-Methyl-1-pentyl, 4-Methyl-1-pentyl, 2-Methyl-2-pentyl, 3-Methyl-2-pentyl, 4-Methyl-2-pentyl, 2-Methyl-3-pentyl, 3-Methyl-3-pentyl, 2,2-Dimethyl-1-butyl, 2,3-Dimethyl-1-butyl, 3,3-Dimethyl-1-butyl, 2-Ethyl-1-butyl, 2,3-Dimethyl-2-butyl, 3,3-Dimethyl-2-butyl, Heptyl, Octyl, Nonyl, Decyl, Undecyl oder Dodecyl. Besonders bevorzugte Methide (Vh) sind [(F₃C-SO₂)₃C]⁻ (Tris(trifluoromethylsulfonyl)methid), [(F₅C₂-SO₂)₃C]⁻ (Bis(pentafluoroethylsulfonyl)methid) und jene, in denen die Reste R^{m} bis R^{o} unabhängig voneinander für Methyl, Ethyl, Propyl, Butyl, Phenyl, Trichlormethyl, Dichlormethyl, Chlormethyl, Trifluormethyl, Difluormethyl oder Fluormethyl stehen.

Handelt es sich bei dem Anion A^{a-} um ein organisches Sulfat (Vi) [R^{p}O-SO₃]⁻, so steht der Rest R^{p} bevorzugt für einen verzweigten oder unverzweigten C₁- bis C₃₀-Alklylrest und besonders bevorzugt für Methylsulfat, Ethylsulfat, Propylsulfat, Butylsulfat, Pentylsulfat, Hexylsulfat, Heptylsulfat oder Octylsulfat.

Handelt es sich bei dem Anion A^{a-} um ein Halometallat (Vj) [M_{q}Halᵣ]^{s-}, so steht M bevorzugt für Aluminium, Zink, Eisen, Cobald, Antimon oder Zinn. Hal steht bevorzugt für Chlor oder Brom und ganz besonders bevorzugt für Chlor. q ist bevorzugt 1, 2 oder 3 und r und s ergeben sich entsprechend der Stöchiometrie und Ladung des Metallions.

Ganz besonders bevorzugt stellt man beim erfindungsgemäßen Verfahren ein gereingtes 1,3-substituierte Imidazoliumsalz (I) her, welches als Anion A^{a-} Tetrafluoroborat, Hexafluorophosphat, Trifluormethansulfonat, Methansulfonat, Formiat, Acetat, Mandelat, Nitrat, Nitrit, Trifluoracetat, Sulfat, Hydrogensulfat, Methylsulfat, Ethylsulfat, Propylsulfat, Butylsulfat, Pentylsulfat, Hexylsulfat, Heptylsulfat, Octylsulfat, Phosphat, Dihydrogenphosphat, Hydrogenphosphat, Propionat, Tetrachloroaluminat, Al₂Cl₇⁻, Chlorozinkat, Chloroferrat, Bis(trifluoromethylsulfonyl)imid (Triflimid), Bis(pentafluoroethylsulfonyl)imid, Tris(trifluoro-methylsulfonyl)methid (Methid), Bis(pentafluoroethylsulfonyl)methid, p-Tolylsulfonat (Tosylat), Bis[salicylato(2-)]borat, Tetracarbonylcobaltat, Dimethylenglykolmonomethylethersulfat, Oleat, Stearat, Acrylat, Methacrylat, Maleinat, Hydrogencitrat, Vinyl-phosphonat, Bis(pentafluoroethyl)phosphinat, Bis[oxalato(2-)]borat, Bis[1,2-benzoldiolato(2-)-O,O']borat, Dicyanamid, Tris(pentafluoroethyl)trifluorophosphat, Tris(heptafluoropropyl)trifluorophosphat, Tetracyanoborat oder Chlorocobaltat enthält.

Ganz besonders bevorzugt stellt man beim erfindungsgemäßen Verfahren als gereingtes 1,3-substituiertes Imidazoliumsalz (I) das Tetrafluoroborat, Hexafluorophosphat, Trifluormethansulfonat, Methansulfonat, Formiat, Acetat, Trifluoracetat, Sulfat, Hydrogensulfat, Methylsulfat, Ethylsulfat, Propylsulfat, Butylsulfat, Pentylsulfat, Hexylsulfat, Heptylsulfat, Octylsulfat, Phosphat, Dihydrogenphosphat, Hydrogenphosphat, Propionat, Tetrachloroaluminat, Al₂Cl₇⁻, Chlorozinkat, Chloroferrat, Bis(trifluoromethyl-sulfonyl)imid, Bis(pentafluoroethyl-sulfonyl)imid, Tris(trifluoromethylsulfonyl)methid, Bis(pentafluoroethylsulfonyl)methid, p-Tolylsulfonat des 1,3-Dimethylimdazoliums, 1-Ethyl-3-methylimidazoliums und 1-Butyl-3-methylimidazoliums her.

Das beim erfindungsgemäßen Verfahren einzusetzende 1,3-substituierte Imidazoliumsalz (II) enthält das 1,3-substituierte Imidazoliumkation (Ia) wie weiter oben definiert und somit identisch ist mit dem 1,3-substituierte Imidazoliumkation (Ia) des herzustellenden gereinigten 1,3-substituierten Imidazoliumsalzes (1), und das Anion Y^{y-}, welches für ein teil- oder volldeprotoniertes Anion einer anorganischen oder organischen Protonensäure H_{y}Y (IV) steht, wobei y eine ganze, positive Zahl ist und den Ladungszustand des Anions wiedergibt. Als Anion Y^{y-} sind prinzipiell alle teil- oder volldeprotoniertes Anionen einer anorganischen oder organischen Protonensäure geeignet, welche mit dem 1,3-substituierten Imidazoliumkation (Ia) Salze bilden. Bevorzugt steht Y^{y-} für ein teil- oder volldeprotoniertes Anion einer anorganischen oder organischen Protonensäure, wie es bei der Defintion des Anions A^{a-} beschrieben ist sowie für Chlorid, Bromid und Iodid.

Besonders bevorzugt setzt man beim erfindungsgemäßen Verfahren ein 1,3-substituiertes Imidazoliumsalz (II) ein, welches als Anion Y^{y-} Chlorid, Bromid, Methansulfonat, Hydrogencarbonat, Carbonat, Hydrogensulfat, Diethylphosphat, Tosylat oder Methylsulfat enthält.

Das einzusetzende 1,3-substituierte Imidazoliumsalz (II) ist beispielsweise durch allgemein bekannte Synthesen, wie etwa durch Alkylierung des entsprechenden 1-substituierten Imidazols zugänglich.

Als starke Base können beim erfindungsgemäßen Verfahren prinzipiell alle Basen eingesetzt werden, welche unter den gegebenen Reaktionsbedingungen wie beispielsweise Druck und Temperatur in der Lage sind, das eingesetzte 1,3-substituierte lmidazoliumsalz (11) zum entsprechenden 1,3-substituierten Imidazol-2-yliden umzusetzen. Als geeignete starke Basen seien beispielsweise Alkoholate, wie etwa die Alkali- und Erdalkalisalze von aliphatischen Alkoholen, Hydroxide wie etwa die Alkali- und Erdalkalihydroxide (z.B. Natrium-, Kalium oder Kalziumhydroxid und deren Kronenetherkomplexe, Oxide wie etwa die Alkali- und Erdalkalioxide (z.B. Natrium-, Kalium oder Kalziumoxid), Carbonate und Hydrogencarbonate wie etwa die Alkali- und Erdalkalivarbonate und -hydrogencarbonate (z.B. Natrium- oder Kaliumcarbonat oder -hydrogencarbonat), Ammoniak, Amine, Metallhydride wie etwa Natrium- oder Kaliumhydrid, Lithiumborhydrid, Lithiumaluminiumhydrid, Butyllithium, Phenolate wie etwa Natrium- oder Kaliumphenolat und deren Kronenetherkomplexe, Amidine, Amide wie etwa Natriumamid, 1,8-Diazabicyclo[5.4.0]undec-7-en (DBU) oder 1,5-Diazabicyclo[4.3.0]non-5-en (DBN) genannt. Bevorzugt setzt man als starke Base Alkali C₁- bis C₆-Alkylate, wie beispielsweise Methylat, Ethylat, n-Butylat, sek.-Butylat, iso-Butylat, tert.-Butylat, Pentylat oder Hexylat ein. Ganz besonders bevorzugt setzt man Natriummetyhlat, Natriumethylat oder Kalium-tert.-butylat ein.

Die molare Menge an zugesetzter starker Base beträgt im Allgemeinen 0,8 bis 5, bevorzugt 0,9 bis 3 und besonders bevorzugt 1,0 bis 1,1, bezogen auf die molare Menge an 1,3-substituiertem Imidazoliumkation (Ia).

Die Umsetzung zwischen dem 1,3-substituierten Imidazoliumsalz (II) und der starken Base zum entsprechendnen 1,3-substituierte Imidazol-2-yliden erfolgt bei einer Temperatur von 20 bis 250°C, bevorzugt von 20 bis 200°C und besonders bevorzugt von 25 bis 150°C. Im Allgemeinen führt man die Umsetzung bei einem Druck von 0,0001 bis 0,15 MPa abs, bevorzugt von 0,0001 bis 0,1 MPa abs und besonders bevorzugt von 0,005 bis 0,05 MPa abs durch.

Die Umsetzung kann prinzipiell in allen Apparaten erfolgen, welche eine entsprechende Vermischung des eingesetzten 1,3-substituierten Imidazoliumsalz (II) und der starken Base erlauben und aus denen die Abdestillation des gebildeten 1,3-substituierte lmidazol-2-ylidens möglich ist. Als Beispiele seien Rührkessel, Dünnschichtverdampfer, Flashverdampfer und Destillationskolonnen genannt.

Kern der vorliegenden Erfindung ist die Maßnahme, dass man das abdestillierte 1,3-substituierte Imidazol-2-yliden im gasförmigen Zustand mit der Protonensäure HₐA (111) in Kontakt bringt und/oder das abdestillierte 1,3-substituierte Imidazol-2-yliden im gasförmigen oder kondensierten Zustand in eine, die Protonensäure HₐA (111) enthaltende Vorlage leitet. Dadurch wird im gesamten Verfahren eine nur kurze Verweilzeit des 1,3-substituierten Imidazol-2-ylidens und insbesondere des flüssigen 1,3-substituierten Imidazol-2-ylidens sichergestellt.

In einer bevorzugten Variante bringt man das abdestillierte 1,3-substituierte Imidazol-2-yliden im gasförmigen Zustand mit flüssiger und besonders bevorzugt gasförmiger Protonensäure HₐA (III) in Kontakt und isoliert das kondensierte, gereinigte 1,3-substituierte Imidazoliumsalz (1). Die Inkontaktbringung kann beispielsweise in einer Destillationskolonne oder einer bevorzugt gepackten Verweilzeitstrecke erfolgen. Das abdestillierte 1,3-substituierte Imidazol-2-yliden und die Protonensäure HₐA-(III) werden dabei bevorzugt im Gegenstrom miteinander in Kontakt gebracht, wobei das gebildete, gereinigte 1,3-substituierte Imidazoliumsalz (I) aufgrund seines hohen Siedepunktes kondensiert und nach unten abgeführt wird, so dass das gereinigte 1,3-substituierte lmidazoliumsalz (I) direkt als gewünschtes Endprodukt zugänglich ist. Als geeignete Reaktionsapparate hierfür seien allgemein Apprate, in denen zwei Gasströme vermischt bzw. in Kontakt gebracht werden können, beispielsweise Destillationskolonnen oder Rohrreaktoren genannt.

In einer anderen bevorzugten Variante leitet man das abdestillierte 1,3-substituierte Imidazol-2-yliden im gasförmigen Zustand in eine, die Protonensäure HₐA (III) enthaltende Vorlage und isoliert daraus das gereinigte 1,3-substituierte Imidazoliumsalz (1). Als geeignete Apparate seien beispielsweise Rührkessel mit Gaseinleitungsvorrichtung, Rührkessel mit Kolonne, Kondensator und Vorlage genannt. Im Allgemeinen ist es von Vorteil, die Vorlage zu kühlen, um einer Verdampfung der vorgelegten Protonensäure (III) entgegenzuwirken. Bei dem gasförmigen 1,3-substituierten Imidazol-2-yliden kann es sich beispielsweise um das gasförmige Kopfprodukt einer Destillationskolonne oder direkt um den gasförmigen Strom aus dem Apparat, in dem die Bildung des 1,3-substituierten Imidazol-2-ylidens erfolgt, handeln. Im Allgemeinen setzt man die Protonensäure (III) gegenüber dem 1,3-substituierten Imidazol-2-yliden in stöchiometrischer oder überstöchiometrischer Menge ein. Überschüssige Protonensäure (III) entfernt man dann nach der Reaktion in einfacher Weise durch Abdestillation oder durch flüssig/flüssig-Extraktion, so dass das gereinigte 1,3-substituierte Imidazoliumsalz (I) direkt als gewünschtes Endprodukt zugänglich ist.

In einer weiteren bevorzugten Variante kondensiert man das abdestillierte 1,3-substituierte Imidazol-2-yliden in einem Kondensor, leitet es im kondensierten Zustand in eine, die Protonensäure HₐA (III) enthaltende Destillationsvorlage und isoliert daraus das gereinigte 1,3-substituierte Imidazoliumsalz (I). Als geeignete Apparate seien beispielsweise Rührkessel mit Einleitungsvorrichtung genannt. Im Allgemeinen ist es von Vorteil, die Vorlage zu kühlen, um einer Verdampfung der vorgelegten Protonensäure (111) entgegenzuwirken. Im Allgemeinen setzt man die Protonensäure (111) gegenüber dem 1,3-substituierten Imidazol-2-yliden in stöchiometrischer oder überstöchiometrischer Menge ein. Überschüssige Protonensäure (111) entfernt man dann nach der Reaktion in einfacher Weise durch Abdestillation oder durch flüssiglflüssig-Extraktion, so dass das gereinigte 1,3-substituierte Imidazoliumsalz (1) direkt als gewünschtes Endprodukt zugänglich ist.

Durch das erfindungsgemäße Verfahren sind die 1,3-substituierten Imidazoliumsalze (I) mit einer besonders hohen Reinheit herstellbar. Sofern nicht das Chlorid-, Bromid- und Iodidsalz als gereinigtes 1,3-substituiertes Imidazoliumsalz (I) gewünscht ist, beträgt die Gesamtkonzentration an Chlorid-, Bromid- und Iodidionen bevorzugt ≤ 100 Gew.-ppm, besonders bevorzugt ≤ 50 Gew.-ppm, ganz besonders bevorzugt ≤ 10 Gew.-ppm und insbesondere ≤ 5 Gew.-ppm. Sofern nicht gereinigtes 1,3-substituiertes Imidazoliumsalz (I) mit einem Metall-haltigen Anion gewünscht ist, beträgt die Metall-Gesamtkonzentration bevorzugt ≤ 100 Gew.-ppm, besonders bevorzugt ≤ 50 Gew.-ppm, ganz besonders bevorzugt ≤ 10 Gew.-ppm und insbesondere ≤ 1 Gew.-ppm. Unter Metall-Gesamtkonzentration ist dabei die Gesamtkonzentration an Metall zu verstehen, welches sich in gebundener oder ungebundener, neutraler oder ionischer Form gelöst oder suspendiert im aufgeschmolzenen 1,3-substituierten Imidazoliumsalz (I) befindet.

Das erfindungsgemäße Verfahren ermöglicht ein Verfahren zur Herstellung von gereinigten 1,3-substituierten Imidazoliumsalzen, welches in Bezug auf die Wahl der Substituenten des Imidazolium-Kations und die Wahl des Anions eine große Variabilität und Flexibilität aufweist und in technisch einfacher Art und Weise mit hoher Ausbeute zu reinen bis hochreinen 1,3-substituierten Imidazoliumsalzen führt. Beim erfindungsgemäßen Verfahren können 1,3-substituierte Imidazoliumsalze eingesetzt werden, welche auf beliebigem Wege hergestellt wurden. Diese können auch diverse Verunreinigungen enthalten. Somit stellt das erfindungsgemäße Verfahren keine nennenswerten Anforderungen an die Qualität des einzusetzenden 1,3-substituierten Imidazoliumsalzes. Durch den Reinigungsschritt über das gasförmige 1,3-substituierte Imidazol-2-yliden verbleiben die ursprünglich vorhandenen Verunreinigungen in der Vorlage. Die anschließende Umsetzung des 1,3-substituierte Imidazol-2-ylidens mit der gewünschten Protonensäure führt somit zu einem besonders reinen Produkt, welches beispielsweise ohne weitere Reinigung für Anwendungen auf dem Elektronik-Gebiet geeignet ist ("electronic grade"). Durch die direkte Umsetzung des gasförmigen 1,3-substituierte Imidazol-2-ylidens mit der gewünschten Protonensäure beziehungsweise die Einleitung des gasförmigen oder kondensierten 1,3-substituierte Imidazol-2-ylidens in eine, die Protonensäure enthaltende Vorlage wird die Bildung etwaiger Neben- oder Zersetzungsprodukte aus dem reaktiven 1,3-substituierte Imidazol-2-yliden verhindert oder zumindest signifikant herabgesetzt.

### Beispiele

### Vergleichsbeispiel 1

7 g (0,04 Mol) [BMIM]Cl wurden mit 3,24 g (0,06 Mol) Natriummethylat gemischt, worauf sich der Ansatz leicht gelblich verfärbte. Das gebildete 1-Butyl-3-methylimidazol-2-yliden wurde anschließend im Vakuum bei einer Übergangstemperatur von etwa 70°C abdestilliert und in einer von außen mit Trockeneis gekühlten Destillationsvorlage aufgefangen. Das erhaltene Destillat wurde sofort ¹H und ¹³C NMR-spektroskopisch analysiert. Dabei konnte kein 1-Butyl-3-methylimidazol-2-yliden nachgewiesen werden. Das Destillat bestand aus nicht weiter identifizierten Zersetzungsprodukten.

### Beispiel 2 (erfindungsgemäß)

22 g (0,15 Mol) 1-Ethyl-3-methylimidazoliumchlorid (im Folgenden als [EMIM]Cl abgekürzt) wurden mit 25,2 g (0,225 Mol) Kalium-tert-butylat gemischt. Das gebildete 1-Ethyl-3-methylimidazol-2-yliden wurde anschließend im Vakuum bei einem Druck von 40 Pa abs und einer Übergangstemperatur von 52-57°C abdestilliert und in eine mit 9,0 g (0,15 Mol) Eisessig (konzentrierte Essigsäure) gefüllte und mit Eiswasser gekühlte Destillationsvorlage geleitet. Die Sumpftemperatur betrug 72-100°C. Beim Eintropfen des kondensierten 1-Ethyl-3-methylimidazol-2-ylidens in die Eisessig enthaltende Destillationsvorlage konnte eine exotherme Reaktion beobachtet werden. Das Gemisch in der Destillationsvorlage verfärbte sich dabei gelb-bräunlich. Zusammen mit dem vorgelegten Eisessig wurden insgesamt 21,7 g Destillat erhalten. Das erhaltene Destillat wurde ¹H und ¹³C NMR-spektroskopisch analysiert. Die Spektren zeigen Signale von [EMIM]-acetat sowie von überschüssiger Essigsäure und tert.-Butanol. Nach Abtrennung des überschüssigen Eisessigs und tert.-Butanols bei 80°C im Vakuum wurden 15,6 g EMIM-acetat erhalten. Das entspricht einer Ausbeute von 61%.

### Beispiel 3 (erfindungsgemäß)

22 g (0,15 Mol) geschmolzenes [EMIM]CI wurden mit 25,2 g (0,225 Mol) Kalium-tert.-butylat gemischt. Das gebildete 1-Ethyl-3-methylimidazol-2-yliden wurde anschließend im Vakuum bei einem Druck von 40-200 Pa abs und einer Übergangstemperatur von 47-52°C abdestilliert und in eine mit 14,4 g (0,15 Mol) Methansulfonsäure gefüllte und mit Eiswasser gekühlte Destillationsvorlage geleitet. Die Sumpftemperatur betrug 75 bis 110°C. Beim Eintropfen des kondensierten 1-Ethyl-3-methylimidazol-2-ylidens in die Methansulfonsäure enthaltende Destillationsvorlage konnte eine exotherme Reaktion beobachtet werden. Das Gemisch in der Destillationsvorlage verfärbte sich dabei gelb-bräunlich. Zusammen mit der vorgelegten Methansulfonsäure wurden insgesamt 28,4 g Destillat erhalten. Das erhaltene Destillat wurde ¹H und ¹³C NMR-spektroskopisch analysiert. Laut NMR-Analyse handelte es sich um [EMIM]-methansulfonat. Die Ausbeute betrug 91,8%.

### Vergleichsbeispiel 4

25 g (0,143 Mol) 1-Butyl-3-methylimidazoliumchlorid (im Folgenden als [BMIM]CI abgekürzt) wurden mit 24,1 g (0,215 Mol) Kalium-tert-butylat gemischt, worauf sich der Ansatz sofort orange-rot verfärbte. Das gebildete 1-Butyl-3-methylimidazol-2-yliden wurde anschließend im Vakuum bei einem Druck von 20 Pa abs und einer Übergangstemperatur von 58-66°C abdestilliert. Die Sumpftemperatur betrug 85-93°C. Es wurden 14,34 g Destillat erhalten und im Anschluß an die Destillation sofort mit Methanol gemischt. Der pH-Wert des Gemisches betrug 12. Die erhaltene Mischung wurde sofort 1H und 13C NMR-spektroskopisch analysiert. Dabei konnte weder 1-Butyl-3-methylimidazol-2-yliden noch das 1-Butyl-3-methylimidazolkation nachgewiesen werden. Der Hauptanteil des Gemisches sind nicht weiter identifizierte Zersetzungsprodukte.

## Patentansprüche

1. Verfahren zur Herstellung von gereinigten 1,3-substituierten Imidazoliumsalzen der allgemeinen Formel (I) 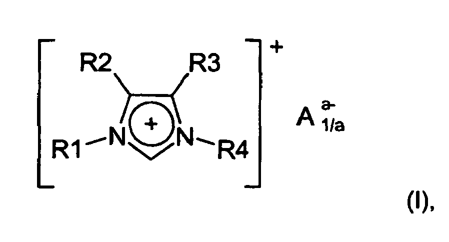 in der
* die Reste R¹, R², R³ und R⁴ unabhängig voneinander einen Kohlenstoff enthaltenden organischen, gesättigten oder ungesättigten, acyclischen oder cyclischen, aliphatischen, aromatischen oder araliphatischen Rest mit 1 bis 30 Kohlenstoffatomen, welcher ein oder mehrere Heteroatome enthalten und/oder durch eine oder mehrere funktionelle Gruppen oder Halogen substituiert sein kann, bedeuten, wobei die jeweils benachbarten Reste R¹ und R², R² und R³ sowie R³ und R⁴ auch miteinander verbunden sein können und die Reste R² und R³ zusätzlich noch unabhängig voneinander Wasserstoff, Halogen oder eine funktionelle Gruppe bedeuten;
und
* A^{a-} für das teil- oder volldeprotonierte Anion einer anorganischen oder organischen Protonensäure HₐA (111) steht, wobei a eine ganze, positive Zahl ist und den Ladungszustand des Anions wiedergibt,
durch Umsetzung eines 1,3-substituierten Imidazoliumsalzes der allgemeinen Formel (II), 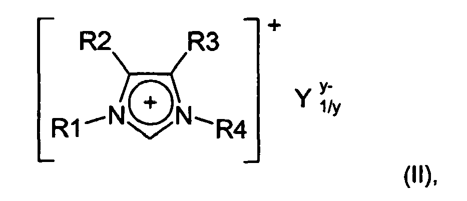 in der die Reste R¹, R², R³ und R⁴ die oben genannte Bedeutung besitzen und das Anion Y^{y-} für das teil- oder volldeprotonierte Anion einer anorganischen oder organischen Protonensäure H_{y}Y (IV) steht, wobei y eine ganze, positive Zahl ist und den Ladungszustand des Anions wiedergibt, mit einer starken Base bei einer Temperatur im Bereich von 20 bis 250°C unter Abdestillation des gebildeten 1,3-substituierten Imidazol-2-ylidens, **dadurch gekennzeichnet, dass** man das abdestillierte 1,3-substituierte Imidazol-2-yliden im gasförmigen Zustand mit der Protonensäure HₐA (III) in Kontakt bringt und/oder das abdestillierte 1,3-substituierte Imidazol-2-yliden im gasförmigen oder kondensierten Zustand in eine, die Protonensäure HₐA (III) enthaltende Vorlage leitet.

2. Verfahren nach Anspruch 1, **dadurch gekennzeichnet, dass** man ein gereinigtes 1,3-substituiertes Imidazoliumsalz (1) herstellt, bei dem die Reste R¹ und R⁴ unabhängig voneinander für Methyl, Ethyl, 1-Propyl, 2-Propyl, 1-Butyl, 1-Pentyl, 1-Hexyl, 1-Heptyl, 1-Octyl, 1-Nonyl, 1-Decyl, 1-Dodecyl, 1-Tetradecyl, 1-Hexadecyl, 1-Octadecyl, 1-(2-Ethyl)hexyl, Benzyl, 3-Phenylpropyl, 6-Hydroxyhexyl oder Phenyl und die Reste R² und R³ unabhängig voneinander für Wasserstoff, Methyl, Ethyl, n-Propyl, 2-Propyl, 1-Butyl, 1-Hexyl, 6-Hydroxyhexyl, Phenyl oder Chlor stehen.

3. Verfahren nach den Ansprüchen 1 bis 2, **dadurch gekennzeichnet, dass** man ein gereinigtes 1,3-substituiertes Imidazoliumsalz (I) herstellt, bei dem das Anion A^{a}für
Fluorid; Hexafluorophosphat; Hexafluoroarsenat; Hexafluoroantimonat; Trifluoroarsenat; Nitrit; Nitrat; Sulfat; Hydrogensulfat; Carbonat; Hydrogencarbonat; Phosphat; Hydrogenphosphat; Dihydrogenphosphat; Vinylphosphonat; Dicyanamid; Bis(pentafluoroethyl)phosphinat; Tris(pentafluoroethyl)trifluorophosphat; Tris(heptafluoropropyl)trifluorophosphat; Bis[oxalato(2-)]borat; Bis[salicylato(2-)]borat; Bis[1,2-benzoldiolato(2-)-O,O']borat; Tetracyanoborat; Tetracarbonylcobaltat;
tetrasubstituiertes Borat der allgemeinen Formel (Va) [BR^{a}R^{b}R^{c}R^{d}]⁻, wobei R^{a} bis R^{d} unabhängig voneinander für Fluor oder einen Kohlenstoff enthaltenden organischen, gesättigten oder ungesättigten, acyclischen oder cyclischen, aliphatischen, aromatischen oder araliphatischen Rest mit 1 bis 30 Kohlenstoffatomen, welcher ein oder mehrere Heteroatome enthalten und/oder durch eine oder mehrere funktionelle Gruppen oder Halogen substituiert sein kann, stehen;
organisches Sulfonat der allgemeinen Formel (Vb) [R^{e}-SO₃]⁻, wobei R^{e} für einen Kohlenstoff enthaltenden organischen, gesättigten oder ungesättigten, acyclischen oder cyclischen, aliphatischen, aromatischen oder araliphatischen Rest mit 1 bis 30 Kohlenstoffatomen, welcher ein oder mehrere Heteroatome enthalten und/oder durch eine oder mehrere funktionelle Gruppen oder Halogen substituiert sein kann, steht;
Carboxylat der allgemeinen Formel (Vc) [R^{f}-COO]⁻, wobei R^{f} für Wasserstoff oder einen Kohlenstoff enthaltenden organischen, gesättigten oder ungesättigten, acyclischen oder cyclischen, aliphatischen, aromatischen oder araliphatischen Rest mit 1 bis 30 Kohlenstoffatomen, welcher ein oder mehrere Heteroatome enthalten und/oder durch eine oder mehrere funktionelle Gruppen oder Halogen substituiert sein kann, steht;
(Fluoralkyl)fluorphosphat der allgemeinen Formel (Vd) [PFₓ(C_{y}F_{2y+1-z}H_{z})₆₋ₓ]⁻, wobei 1 ≤ x ≤ 6, 1 ≤ y ≤ 8 und 0 ≤ z ≤ 2y+1;
Imid der allgemeinen Formeln (Ve) [R^{g}-SO₂-N-SO₂-R^{h}]⁻, (Vf) [Rⁱ-SO₂-N-CO-R^{j}]oder (IVg) [R^{k}-CO-N-CO-R^{l}]-, wobei R^{g} bis R^{l} unabhängig voneinander für Wasserstoff oder einen Kohlenstoff enthaltenden organischen, gesättigten oder ungesättigten, acyclischen oder cyclischen, aliphatischen, aromatischen oder araliphatischen Rest mit 1 bis 30 Kohlenstoffatomen, welcher ein oder mehrere Heteroatome enthalten und/oder durch eine oder mehrere funktionelle Gruppen oder Halogen substituiert sein kann, stehen;
Methid der allgemeinen Formel (Vh) 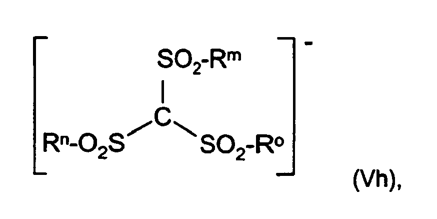 wobei R^{m} bis R^{o} unabhängig voneinander für Wasserstoff oder einen Kohlenstoff enthaltenden organischen, gesättigten oder ungesättigten, acyclischen oder cyclischen, aliphatischen, aromatischen oder araliphatischen Rest mit 1 bis 30 Kohlenstoffatomen, welcher ein oder mehrere Heteroatome enthalten und/oder durch eine oder mehrere funktionelle Gruppen oder Halogen substituiert sein kann, stehen;
organisches Sulfat der allgemeinen Formel (Vi) [R^{p}O-SO₃]⁻, wobei R^{p} für einen Kohlenstoff enthaltenden organischen, gesättigten oder ungesättigten, acyclischen oder cyclischen, aliphatischen, aromatischen oder araliphatischen Rest mit 1 bis 30 Kohlenstoffatomen, welcher ein oder mehrere Heteroatome enthalten und/oder durch eine oder mehrere funktionelle Gruppen oder Halogen substituiert sein kann, steht; oder
Halometallat der allgemeinen Formel (Vj) [M_{q}Halᵣ]^{s-}, wobei M für ein Metall und Hal für Fluor, Chlor, Brom oder Iod steht, q und r ganze positive Zahlen sind und die Stöchiometrie des Komplexes angeben und s eine ganze positive Zahl ist und die Ladung des Komplexes angibt;
steht.

4. Verfahren nach den Ansprüchen 1 bis 3, **dadurch gekennzeichnet, dass** man ein gereinigtes 1,3-substituiertes Imidazoliumsalz (1) herstellt, bei dem das Anion A^{a}für Tetrafluoroborat, Hexafluorophosphat, Trifluormethansulfonat, Methansulfonat, Formiat, Acetat, Mandelat, Nitrat, Nitrit, Trifluoracetat, Sulfat, Hydrogensulfat, Methylsulfat, Ethylsulfat, Propylsulfat, Butylsulfat, Pentylsulfat, Hexylsulfat, Heptylsulfat, Octylsulfat, Phosphat, Dihydrogenphosphat, Hydrogenphosphat, Propionat, Tetrachloroaluminat, Al₂Cl₇⁻, Chlorozinkat, Chloroferrat, Bis(trifluoromethylsulfonyl)imid, Bis(pentafluoroethylsulfonyl)imid, Tris(trifluoromethylsulfonyl)-methid, Bis(pentafluoroethylsulfonyl)methid, p-Tolylsulfonat, Bis[salicylato(2-)]borat, Tetracarbonylcobaltat, Dimethylenglykolmonomethylethersulfat, Octylsulfat, Oleat, Stearat, Acrylat, Methacrylat, Maleinat, Hydrogencitrat, Vnylphosphonat, Bis(pentafluoroethyl)phosphinat, Bis[oxatato(2-)]borat, Bis[1,2-benzoldiolato(2-)-O,O']borat, Dicyanamid, Tris(pentafluoroethyl)trifluorophosphat, Tris(heptafluoropropyl)trifluorophosphat, Tetracyanoborat oder Chlorocobaltat steht.

5. Verfahren nach den Ansprüchen 1 bis 4, **dadurch gekennzeichnet, dass** man ein 1,3-substituiertes Imidazoliumsalz (II) einsetzt, bei dem das Anion Y^{y-} für Chlorid, Bromid, Methansulfonat, Hydrogencarbonat, Carbonat, Hydrogensulfat, Diethylphosphat, Tosylat oder Methylsulfat steht.

6. Verfahren nach den Ansprüchen 1 bis 5, **dadurch gekennzeichnet, dass** man das abdestillierte 1,3-substituierte Imidazol-2-yliden im gasförmigen Zustand mit gasförmiger Protonensäure HₐA (111) in Kontakt bringt und das kondensierte, gereinigte 1,3-substituierte Imidazoliumsalz (1) isoliert.

7. Verfahren nach den Ansprüchen 1 bis 5, **dadurch gekennzeichnet, dass** man das abdestillierte 1,3-substituierte Imidazol-2-yliden im gasförmigen Zustand in eine, die Protonensäure HₐA (III) enthaltende Vorlage leitet und daraus das gereinigte 1,3-substituierte Imidazoliumsalz (I) isoliert.

8. Verfahren nach den Ansprüchen 1 bis 5, **dadurch gekennzeichnet, dass** man das abdestillierte 1,3-substituierte Imidazol-2-yliden in einem Kondensor kondensiert, im kondensierten Zustand in eine, die Protonensäure HₐA (III) enthaltende Destillationsvorlage leitet und daraus das gereinigte 1,3-substituierte Imidazoliumsalz (I) isoliert.

9. Verfahren nach den Ansprüchen 1 bis 8, **dadurch gekennzeichnet, dass** man die Abdestillation bei einem Druck von 0,0001 bis 0,15 MPa abs durchführt.

## Claims

1. A process for preparing purified 1,3-substituted imidazolium salts of the formula (I) 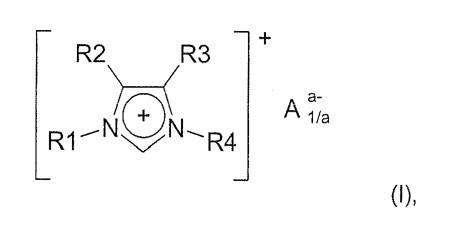 where
* the radicals R¹, R², R³ and R⁴ are each, independently of one another, a carbon-comprising organic, saturated or unsaturated, acyclic or cyclic, aliphatic, aromatic or araliphatic radical which has from 1 to 30 carbon atoms and may comprise one or more heteroatoms and/or be substituted by one or more functional groups or halogens, where adjacent radicals R¹ and R², R² and R³ or R³ and R⁴ may also be joined to one another and the radicals R² and R³ may each also be, independently of one another, hydrogen, halogen or a functional group;
and
* A^{a-} is the partly or fully deprotonated anion of an inorganic or organic protic acid HₐA (III), where a is a positive integer and indicates the charge on the anion,
by reacting a 1,3-substituted imidazolium salt of the formula (II), 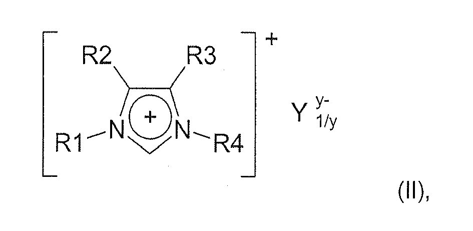 where the radicals R¹, R², R³ and R⁴ are as defined above and the anion Y^{y-} is the partly or fully deprotonated anion of an inorganic or organic protic acid H_{y}Y (IV), where y is a positive integer and indicates the charge on the anion, with a strong base at from 20 to 250°C while distilling off the 1,3-substituted imidazol-2-ylidene formed, wherein the 1,3-substituted imidazol-2-ylidene which has been distilled off is brought into contact in the gaseous state with the protic acid HₐA (III) and/or the 1,3-substituted imidazol-2-ylidene which has been distilled off is passed in the gaseous or condensed state into a receiver comprising the protic acid HₐA (111).

2. The process according to claim 1, wherein a purified 1,3-substituted imidazolium salt (I) in which the radials R¹ and R⁴ are each, independently of one another, methyl, ethyl, 1-propyl, 2-propyl, 1-butyl, 1-pentyl, 1-hexyl, 1-heptyl, 1-octyl, 1-nonyl, 1-decyl, 1-dodecyl, 1-tetradecyl, 1-hexadecyl, 1-octadecyl, 1-(2-ethyl)hexyl, benzyl, 3-phenylpropyl, 6-hydroxyhexyl or phenyl and the radicals R² and R³ are each, independently of one another, hydrogen, methyl, ethyl, n-propyl, 2-propyl, 1-butyl, 1-hexyl, 6-hydroxyhexyl, phenyl or chlorine is prepared.

3. The process according to claim 1 or 2, wherein a purified 1,3-substituted imidazolium salt (I) in which the anion A^{a-} is
fluoride; hexafluorophosphate; hexafluoroarsenate; hexafluoroantimonate; trifluoroarsenate; nitrite; nitrate; sulfate; hydrogensulfate; carbonate; hydrogencarbonate; phosphate; hydrogenphosphate; dihydrogenphosphate; vinyl phosphonate; dicyanamide; bis(pentafluoroethyl)phosphinate; tris(pentafluoroethyl)trifluorophosphate; tris(heptafluoropropyl)trifluorophosphate; bis[oxalato(2-)]borate; bis[salicylato(2-)]borate; bis[1,2-benzenediolato(2-)O,O']borate; tetracyanoborate; tetracarbonylcobaltate;
tetrasubstituted borate of the formula (Va) [BR^{a}R^{b}R^{c}R^{d}]⁻, where R^{a} to R^{d} are each, independently of one another, fluorine or a carbon-comprising organic, saturated or unsaturated, acyclic or cyclic, aliphatic, aromatic or araliphatic radical which has from 1 to 30 carbon atoms and may comprise one or more heteroatoms and/or be substituted by one or more functional groups or halogens;
organic sulfonate of the formula (Vb) [R^{e}-SO₃]-, where R^{e} is a carbon-comprising organic, saturated or unsaturated, acyclic or cyclic, aliphatic, aromatic or araliphatic radical which has from 1 to 30 carbon atoms and may comprise one or more heteroatoms and/or be substituted by one or more functional groups or halogens;
carboxylate of the formula (Vc) [R^{f}-COO]⁻, where R^{f} is hydrogen or a carbon-comprising organic, saturated or unsaturated, acyclic or cyclic, aliphatic, aromatic or araliphatic radical which has from 1 to 30 carbon atoms and may comprise one or more heteroatoms and/or be substituted by one or more functional groups or halogens;
(fluoroalkyl)fluorophosphates of the formula (Vd) [PFₓ(C_{y}F_{2y+1-z}H_{z})₆₋ₓ]⁻, where 1 ≤ x ≤ 6, 1 ≤ y ≤ 8 and 0 ≤ z ≤ 2y+1;
imide of the formula (Ve) [R^{g}-SO₂-N-SO₂-R^{h}]⁻, (Vf) [Rⁱ-SO₂-N-CO-R^{j}]⁻ or (IVg) [R^{k}-CO-N-CO-R^{l}]⁻, where R^{g} to R^{l} are each, independently of one another, hydrogen or a carbon-comprising organic, saturated or unsaturated, acyclic or cyclic, aliphatic, aromatic or araliphatic radical which has from 1 to 30 carbon atoms and may comprise one or more heteroatoms and/or be substituted by one or more functional groups or halogens;
methide of the formula (Vh) 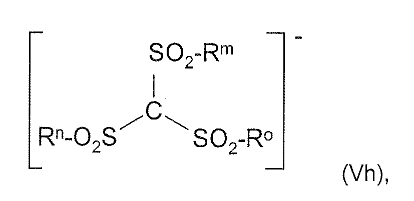 where R^{m} to R^{o} are each, independently of one another, hydrogen or a carbon-comprising organic, saturated or unsaturated, acyclic or cyclic, aliphatic, aromatic or araliphatic radical which has from 1 to 30 carbon atoms and may comprise one or more heteroatoms and/or be substituted by one or more functional groups or halogens;
organic sulfate of the formula (Vi) [R^{p}O-SO₃]⁻, where R^{p} is a carbon-comprising organic, saturated or unsaturated, acyclic or cyclic, aliphatic, aromatic or araliphatic radical which has from 1 to 30 carbon atoms and may comprise one or more heteroatoms and/or be substituted by one or more functional groups or halogens; or
halometalate of the formula (Vj) [M_{q}Halᵣ]^{s-}, where M is a metal and Hal is fluorine, chlorine, bromine or iodine, q and r are positive integers and indicate the stoichiometry of the complex and s is a positive integer and indicates the charge on the complex;
is prepared.

4. The process according to any of claims 1 to 3, wherein a purified 1,3-substituted imidazolium salt (I) in which the anion A^{a-} is tetrafluoroborate, hexafluorophosphate, trifluoromethanesulfonate, methanesulfonate, formate, acetate, mandelate, nitrate, nitrite, trifluoroacetate, sulfate, hydrogensulfate, methyl sulfate, ethyl sulfate, propyl sulfate, butyl sulfate, pentyl sulfate, hexyl sulfate, heptyl sulfate, octyl sulfate, phosphate, dihydrogenphosphate, hydrogenphosphate, propionate, tetrachloroaluminate, Al₂Cl₇⁻, chlorozincate, chloroferrate, bis(trifluoromethylsulfonyl)imide, bis(pentafluoroethylsulfonyl)imide, tris(trifluoromethylsulfonyl)methide, bis(pentafluoroethylsulfonyl)methide, p-toluenesulfonate, bis[salicylato(2-)]borate, tetracarbonylcobaltate, dimethylene glycol monomethyl ether sulfate, octyl sulfate, oleate, stearate, acrylate, methacrylate, maleate, hydrogencitrate, vinyl phosphonate, bis(pentafluoroethyl)phosphinate, bis[oxalato(2-)]borate, bis[1,2-benzenediolato(2-)O,O']borate, dicyanamide, tris(pentafluoroethyl)trifluorophasphate, tris(heptafluoropropyl)trifluorophosphate, tetracyanoborate or chlorocobaltate is prepared.

5. The process according to any of claims 1 to 4, wherein a 1,3-substituted imidazolium salt (II) in which the anion Y^{y-} is chloride, bromide, methanesulfonate, hydrogencarbonate, carbonate, hydrogensulfate, diethylphosphate, tosylate or methyl sulfate is prepared.

6. The process according to any of claims 1 to 5, wherein the 1,3-substituted imidazol-2-ylidene which has been distilled off is brought into contact in the gaseous state with gaseous protic acid HₐA (III) and the condensed, purified 1,3-substituted imidazolium salt (I) is isolated.

7. The process according to any of claims 1 to 5, wherein the 1,3-substituted imidazol-2-ylidene which has been distilled off is passed in the gaseous state into a receiver comprising the protic acid HₐA (III) and the purified 1,3-substituted imidazolium salt (I) is isolated therefrom.

8. The process according to any of claims 1 to 5, wherein the 1,3-substituted imidazol-2-ylidene which has been distilled off is condensed in a condenser, passed in the condensed state into a distillation receiver comprising the protic acid HₐA (III) and the purified 1,3-substituted imidazolium salt (I) is isolated therefrom.

9. The process according to any of claims 1 to 8, wherein the distillation is carried out at a pressure of from 0.0001 to 0.15 MPa abs.

## Revendications

1. Procédé de préparation de sels d'imidazolium substitués en position 1,3 purifiés de la formule générale (I) : 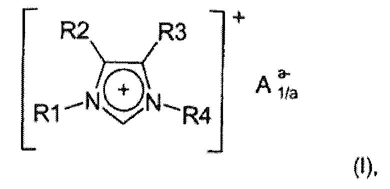 dans laquelle
* les radicaux R¹, R², R³ et R⁴ représentent, indépendamment les uns des autres, un radical organique contenant du carbone, saturé ou insaturé, acyclique ou cyclique, aliphatique, aromatique ou araliphatique, comportant 1 à 30 atomes de carbone, qui peut contenir un ou plusieurs hétéroatomes et/ou être substitué par un ou plusieurs groupes fonctionnels ou de l'halogène, les radicaux respectivement voisins R¹ et R², R² et R³ ainsi que R³ et R⁴ pouvant aussi être reliés l'un à l'autre et les radicaux R² et R³ signifiant en supplément encore, indépendamment l'un de l'autre, de l'hydrogène, de l'halogène ou un groupe fonctionnel,
et
* A^{a-} représente l'anion partiellement ou totalement déprotoné d'un acide protonique inorganique ou organique HₐA (III), a étant un nombre entier positif et représentant l'état de charge de l'anion,
par réaction d'un sel d'imidazolium substitué en position 1,3 de la formule générale (II) : 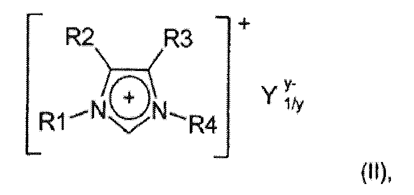 dans laquelle les radicaux R¹, R², R³ et R⁴ ont la signification donnée ci-dessus et l'anion Y^{y}représente l'anion totalement ou partiellement déprotoné d'un acide protonique inorganique ou organique H_{y}Y (IV), y étant un nombre entier positif et représentant l'état de charge de l'anion, avec une base forte à une température de l'ordre de 20 à 250°C, avec séparation par distillation de l'imidazol-2-ylidène substitué en position 1,3 formé, **caractérisé en ce qu'**on met en contact l'imidazol-2-ylidène substitué en position 1,3 distillé, à l'état gazeux, avec l'acide protonique HₐA (III) et/ou on conduit l'imidazol-2-ylidène substitué en position 1,3 distillé, à l'état gazeux ou condensé dans un récipient contenant l'acide protonique HₐA (III).

2. Procédé suivant la revendication 1, **caractérisé en ce qu'**on prépare un sel d'imidazolium substitué en position 1,3 purifié (I) dans lequel les radicaux R¹ et R⁴ représentent, indépendamment l'un de l'autre, un groupe méthyle, éthyle, 1-propyle, 2-propyle, 1-butyle, 1-pentyle, 1-hexyle, 1-heptyle, 1-octyle, 1-nonyle, 1-décyle, 1-dodécyle, 1-tétradécyle, 1-hexadécyle, 1-octadécyle, 1-(2-éthyl)hexyle, benzyle, 3-phénylpropyle, 6-hydroxyhexyle ou phényle et les radicaux R² et R³ représentent, indépendamment l'un de l'autre, de l'hydrogène, du chlore ou un groupe méthyle, éthyle, n-propyle, 2-propyle, 1-butyle, 1-hexyle, 6-hydroxyhexyle ou phényle.

3. Procédé suivant les revendications 1 à 2, **caractérisé en ce qu'**on prépare un sel d'imidazolium substitué en position 1,3, purifié (I), dans lequel l'anion A^{a-} représente
du fluorure, de l'hexafluorophosphate, de l'hexafluoroarséniate, de l'hexafluoroantimoniate, du trifluoroarséniate, du nitrite, du nitrate, du sulfate, de l'hydrogénosulfate, du carbonate, de l'hydrogénocarbonate, du phosphate, de l'hydrogénophosphate, du dihydrogénophosphate, du vinylphosphonate, du dicyanamide, du bis-(pentafluoroéthyl)-phosphinate, du tris-(pentafluoroéthyl)-trifluorophosphate, du tris-(heptafluoropropyl)-trifluorophosphate, du bis-[oxalato(2-)]-borate, du bis-[salicylato(2-)]-borate, du bis-[1,2-benzènediolato(2-)-O,O']-borate, du tétracyanoborate, du tétracarbonylcobaltate,
du borate tétrasubstitué de la formule générale (Va) [BR^{a}R^{b}R^{c}R^{d}]⁻, où R^{a} à R^{d} représentent, indépendamment les uns des autres, du fluor ou un radical organique contenant du carbone, saturé ou insaturé, acyclique ou cyclique, aliphatique, aromatique ou araliphatique, comportant 1 à 30 atomes de carbone, qui peut contenir un ou plusieurs hétéroatomes et/ou être substitué par un ou plusieurs groupes fonctionnels ou de l'halogène,
du sulfonate organique de la formule générale (Vb) [R^{e}-SO₃]⁻, où R^{e} représente un radical organique contenant du carbone, saturé ou insaturé, acyclique ou cyclique, aliphatique, aromatique ou araliphatique, comportant 1 à 30 atomes de carbone, qui peut contenir un ou plusieurs hétéroatomes et/ou être substitué par un ou plusieurs groupes fonctionnels ou de l'halogène,
du carboxylate de la formule générale (Vc) [R^{f}-COO]⁻, où R^{f} représente de l'hydrogène ou un radical organique contenant du carbone, saturé ou insaturé, acyclique ou cyclique, aliphatique, aromatique ou araliphatique, comportant 1 à 30 atomes de carbone, qui peut contenir un ou plusieurs hétéroatomes et/ou être substitué par un ou plusieurs groupes fonctionnels ou de l'halogène,
du (fluoroalkyl)fluorophosphate de la formule générale (Vd) [PFₓ(C_{y}F_{2y+1-z}H_{z})₆₋ₓ]⁻ où 1 ≤ x ≤ 6, 1 ≤ y ≤ 8 et 0 ≤ z ≤ 2y+1,
de l'imide des formules générales (Ve) [R^{g}-SO₂-N-SO₂-R^{h}]⁻, (Vf) [Rⁱ-SO₂-N-CO-R^{j}]⁻ ou (IVg) [R^{k}-CO-N-CO-R^{l}]⁻, où R^{g} à R^{l} représentent, indépendamment les uns des autres, de l'hydrogène ou un radical organique contenant du carbone, saturé ou insaturé, acyclique ou cyclique, aliphatique, aromatique ou araliphatique, comportant 1 à 30 atomes de carbone, qui peut contenir un ou plusieurs hétéroatomes et/ou être substitué par un ou plusieurs groupes fonctionnels ou de l'halogène,
du méthide de la formule générale (Vh) : 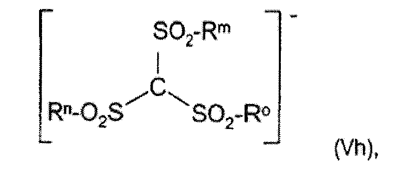 où R^{m} à R^{o} représentent, indépendamment les uns des autres, de l'hydrogène ou un radical organique contenant du carbone, saturé ou insaturé, acyclique ou cyclique, aliphatique, aromatique ou araliphatique, comportant 1 à 30 atomes de carbone, qui peut contenir un ou plusieurs hétéroatomes et/ou être substitué par un ou plusieurs groupes fonctionnels ou de l'halogène,
du sulfate organique de la formule générale (Vi) [R^{p}O-SO₃]⁻, où R^{p} représente un radical organique contenant du carbone, saturé ou insaturé, acyclique ou cyclique, aliphatique, aromatique ou araliphatique, comportant 1 à 30 atomes de carbone, qui peut contenir un ou plusieurs hétéroatomes et/ou être substitué par un ou plusieurs groupes fonctionnels ou de l'halogène, ou
du métallate halogéné de la formule générale (Vj) [MqHalᵣ]^{s-}, où M représente un métal et Hal du fluor, du chlore, du brome ou de l'iode, q et r sont des nombres entiers positifs et indiquent la stoechiométrie du complexe et s est un nombre entier positif et indique la charge du complexe.

4. Procédé suivant les revendications 1 à 3, **caractérisé en ce qu'**on prépare un sel d'imidazolium substitué en position 1,3 purifié (I), dans lequel l'anion A^{a-} représente du tétrafluoroborate, de l'hexafluorophosphate, du trifluorométhanesulfonate, du méthanesulfonate, du formiate, de l'acétate, du mandélate, du nitrate, du nitrite, du trifluoroacétate, du sulfate, de l'hydrogénosulfate, du méthylsulfate, de l'éthylsulfate, du propylsulfate, du butylsulfate, du pentylsulfate, de l'hexylsulfate, de l'heptylsulfate, de l'octylsulfate, du phosphate, du dihydrogénophosphate, de l'hydrogénophosphate, du propionate, du tétrachloroaluminate, du Al₂Cl₇⁻, du chlorozincate, du chloroferrate, du bis-(trifluorométhylsulfonyl)-imide, du bis-(pentafluoroéthylsulfonyl)-imide, du tris-(trifluorométhylsulfonyl)-méthide, du bis-(pentafluoroéthylsulfonyl)-méthide, du p-tolylsulfonate, du bis-[salicylato(2-)]-borate, du tétracarbonylcobaltate, du diméthylèneglycolmonométhyléthersulfate, de l'octylsulfate, de l'oléate, du stéarate, de l'acrylate, du méthacrylate, du maléinate, de l'hydrogénocitrate, du vinylphosphonate, du bis-(pentafluoroéthyl)-phosphinate, du bis-[oxalato(2-)]-borate, du bis-[1,2-benzènediolato(2-)-0,0']-borate, du dicyanamide, du tris-(pentafluoroéthyl)-trifluorophosphate, du tris-(heptafluoropropyl)-trifluorophosphate, du tétracyanoborate ou du chlorocobaltate.

5. Procédé suivant les revendications 1 à 4, **caractérisé en ce qu'**on met en oeuvre un sel d'imidazolium substitué en position 1,3 (II) dans lequel l'anion Y^{y-} représente du chlorure, du bromure, du méthanesulfonate, de l'hydrogénocarbonate, du carbonate, de l'hydrogénosulfate, du diéthylphosphate, du tosylate ou du méthylsulfate.

6. Procédé suivant les revendications 1 à 5, **caractérisé en ce qu'**on met en contact l'imidazol-2-ylidène substitué en position 1,3, distillé, à l'état gazeux, avec de l'acide protonique gazeux HₐA (III) et on isole le sel d'imidazolium substitué en position 1,3 purifié (I), condensé.

7. Procédé suivant les revendications 1 à 5, **caractérisé en ce qu'**on conduit l'imidazol-2-ylidène substitué en position 1,3, distillé, à l'état gazeux, dans un récipient contenant l'acide protonique HₐA (III) et on en isole le sel d'imidazolium substitué en position 1,3 purifié (I).

8. Procédé suivant les revendications 1 à 5, **caractérisé en ce qu'**on condense l'imidazol-2-ylidène substitué en position 1,3, distillé, dans un condenseur, on le conduit à l'état condensé dans un récipient de distillation contenant l'acide protonique HₐA (III) et à partir de là on isole le sel d'imidazolium substitué en position 1,3 purifié (I).

9. Procédé suivant les revendications 1 à 8, **caractérisé en ce qu'**on effectue la séparation par distillation à une pression de 0,0001 à 0,15 MPa abs.
